# EUROPEAN PATENT APPLICATION

(11) **EP 4 763 176 A1**
(43) Date of publication of application: **24.06.2026**
(21) Application number: 24913309.1
(22) Date of filing: 15.10.2024
(51) Int. Cl.: A61H 1/02, A61H 1/00, B25J 9/00, B25J 9/16, A61H 3/00

(54) **WEARABLE DEVICE FOR EVALUATING USER'S SITTING POSTURE AND PROVIDING EXTERNAL FORCE TO USER, AND METHOD FOR OPERATING SAME**

(30) Priority: 28.12.2023 KR 20230195705; 28.02.2024 KR 20240029103
(71) Applicant: Samsung Electronics Co., Ltd., Suwon-si, Gyeonggi-do 16677 (KR)
(72) Inventor: LEE, Hwangjae, Suwon-si Gyeonggi-do 16677 (KR); PARK, Bola, Suwon-si Gyeonggi-do 16677 (KR); SONG, Sukhoon, Suwon-si Gyeonggi-do 16677 (KR); LEE, Dokwan, Suwon-si Gyeonggi-do 16677 (KR); LEE, Hyukchan, Suwon-si Gyeonggi-do 16677 (KR); CHOI, Daehan, Suwon-si Gyeonggi-do 16677 (KR); KIM, Dongwoo, Suwon-si Gyeonggi-do 16677 (KR)
(74) Representative: Gulde & Partner
(86) International application number: PCT/KR2024/015576
(87) International publication number: WO 2025/143468

(57) **Abstract**

A wearable device is disclosed. The wearable device may comprise: a driving module; a first frame corresponding to a portion of a user's lower body; an angle sensor that senses an angle of the first frame to obtain a first frame angle value; a waist support module connected to the driving module and positioned at a waist portion of the user; an IMU sensor; and at least one processor. The at least one processor may obtain the first frame angle value using the angle sensor, obtain a first rotation angle value of the waist support module using the IMU sensor, determine whether a sitting posture of the user is abnormal on the basis of the first rotation angle value and the first frame angle value, and instruct the driving module to generate a first torque when the sitting posture is determined to be abnormal.

## Description

### Technical Field

Certain example embodiments relate to a wearable apparatus for evaluating a sitting posture of a user and/or providing an external force to the user, and/or a method of operating the wearable apparatus.

### Background Art

In general, a walking assistance device refers to a mechanism or device that helps a patient, who for example cannot walk on their own due to various diseases, accidents, and/or the like, or that helps any other type of person, to perform walking exercises such as for rehabilitation treatment or the like. With the recent intense aging of societies, a growing number of people experience inconvenience in walking or have difficulty in normal walking due to malfunctioning joint issues, and there is increasing interest in walking assistance devices. A walking assistance device can be worn on the body of a user to assist the user with walking and/or exercise by providing muscular strength and/or to induce the user to walk in a normal walking pattern.

### Disclosure of the Invention

### Technical Goals

According to an example embodiment, a wearable apparatus may include a driving module comprising a motor and/or circuitry, a first frame corresponding to a portion of a lower body of a user, an angle sensor configured to sense an angle of the first frame and obtain a first frame angle value, a lumbar support module, comprising a lumbar support, connected directly or indirectly to the driving module and configured to be positioned on and/or to support a lower back area of the user, an inertial measurement unit (IMU) sensor, and at least one processor comprising processing circuitry. The processor(s) may be configured to obtain the first frame angle value using the angle sensor, obtain a first rotation angle value of the lumbar support module using the IMU sensor, determine whether a sitting posture of the user is an abnormal sitting posture based on the first rotation angle value and the first frame angle value, and control the driving module such that the driving module generates a first torque when determining that the sitting posture is the abnormal sitting posture.

According to an example embodiment, the lumbar support module may be configured to provide an external force to the user by performing a rotational motion based on the generated first torque.

According to an example embodiment, a method of operating a wearable apparatus may include obtaining a first frame angle value by sensing an angle of a first frame corresponding to a portion of a lower body of a user, obtaining a first rotation angle value of a lumbar support module of the wearable apparatus, determining whether a sitting posture of the user is an abnormal sitting posture based on the first rotation angle value and the first frame angle value, generating a first torque through a driving module of the wearable apparatus when it is determined that the sitting posture is the abnormal sitting posture, and providing an external force to the user by rotating the lumbar support module connected to the driving module based on the generated first torque.

According to an example embodiment, the wearable apparatus may evaluate or determine whether the sitting posture of the user is a normal sitting posture or the abnormal sitting posture.

According to an example embodiment, when the sitting posture of the user is the abnormal sitting posture, the wearable apparatus may provide an external force to a user to correct the sitting posture of the user.

According to an example embodiment, the wearable apparatus may provide a mode in which lower back muscle strength of the user in the sitting posture may be increased.

### Brief Description of the Drawings

The above and other aspects, features, and advantages of certain example embodiments will be more apparent from the following detailed description, taken in conjunction with the accompanying drawings, in which:
FIG. 1A is a diagram illustrating an overview of a wearable apparatus worn on a body of a user, according to an example embodiment.
FIG. 1B is a diagram illustrating an example of a system including a wearable apparatus, according to an example embodiment.
FIG. 2A is a rear schematic view of a wearable apparatus according to an example embodiment.
FIG. 2B is a left side view of a wearable apparatus according to an example embodiment.
FIGS. 3A and 3B are block diagrams illustrating examples of a configuration of a wearable apparatus, according to an example embodiment.
FIG. 4 is a diagram illustrating an interaction between a wearable apparatus and an electronic device, according to an example embodiment.
FIG. 5 is a block diagram illustrating an example of a configuration of a wearable apparatus, according to an example embodiment.
FIG. 6 is a diagram illustrating an example of a rotation angle of a wearable apparatus, according to an embodiment.
FIGS. 7A, 7B, 8A, and 8B are diagrams illustrating examples of an operation in which a wearable apparatus evaluates a sitting posture of a user, according to an example embodiment(s).
FIGS. 9 and 10 are diagrams illustrating examples of an operation in which a wearable apparatus rotates a lumbar support module by generating a first torque, according to an example embodiment.
FIGS. 11 and 12 are diagrams illustrating examples of an operation in which a wearable apparatus rotates the lumbar support module by generating a second torque, according to an example embodiment.
FIGS. 13 and 14 are diagrams illustrating examples of an operation in which a wearable apparatus rotates the lumbar support module by alternately generating the first torque and the second torque, according to an example embodiment.
FIG. 15 is a diagram illustrating an example of an operation in which a wearable apparatus provides a notification, according to an example embodiment.
FIG. 16 is a flowchart illustrating an example of a method of operating a wearable apparatus, according to an example embodiment.

### Best Mode for Carrying Out the Invention

The following detailed structural or functional description is provided as an example only and various alterations and modifications may be made to embodiments. Accordingly, the embodiments are not construed as limited to the disclosure and should be understood to include all changes, equivalents, and replacements within the idea and the technical scope of the disclosure.

Although terms, such as first, second, and the like are used to describe various components, the components are not limited to the terms. These terms should be used only to distinguish one component from another component. For example, a first component may be referred to as a second component, and similarly the second component may also be referred to as the first component.

It should be noted that if one component is described as being "connected", "coupled", or "joined" to another component, at least a third component(s) may be "connected", "coupled", and "joined" between the first and second components, although the first component may be directly connected, coupled, or joined to the second component. Thus, for example, "connected" as used herein covers both direct and indirect connections.

The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, components, or groups thereof, but do not preclude the presence or addition of one or more other features, integers, steps, operations, elements, components, or groups thereof.

Unless otherwise defined, all terms, including technical and scientific terms, used herein have the same meaning as commonly understood by one of ordinary skill in the art to which this disclosure pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Hereinafter, certain example embodiments will be described in detail with reference to the accompanying drawings. When describing the embodiments with reference to the accompanying drawings, like reference numerals refer to like elements and a repeated description related thereto will be omitted.

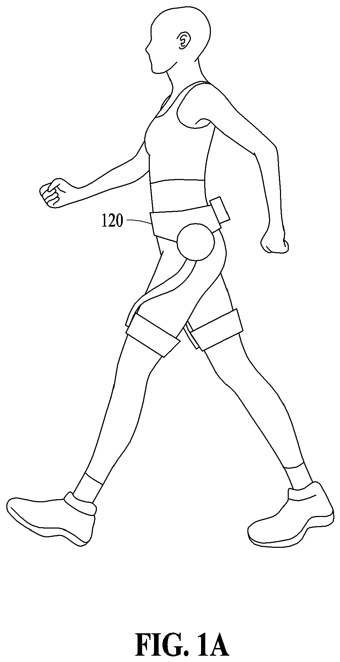

FIG. 1A is a diagram illustrating an overview of a wearable apparatus worn on a body of a user, according to an embodiment.

Referring to FIG. 1A, a wearable apparatus 120 may be a device worn on the body of a user to assist the user in walking, exercising, and/or working. In embodiments, the term "wearable apparatus" may be replaced with "wearable robot," "walking assistance device," or "exercise assistance device". The user may be a human or an animal but is not limited thereto. The wearable apparatus 120 may be worn on the body (e.g., the lower body (the legs, ankles, knees, etc.), the upper body (the torso, arms, wrists, etc.), or the waist) of the user to provide an external force (e.g., an assistance force and/or a resistance force) to a body motion of the user. The assistance force may be a force applied in the same direction as the body motion direction of the user, and the resistance force may be a force applied in a direction opposite to the body motion direction of the user. The term "resistance force" may also be referred to as "exercise load".

When the wearable apparatus 120 performs a walking assist function to assist the user in walking, the wearable apparatus 120 may assist a portion or entirety of a leg of the user by providing an assistance force to the body of the user, thereby assisting the user in walking. The wearable apparatus 120 may enable the user to walk independently or to walk for a long time by providing a force required for the user to walk, thereby extending the walking ability of the user. The wearable apparatus 120 may help in improving an abnormal walking habit or walking posture of a walker.

When the wearable apparatus 120 performs an exercise function to enhance the exercise effect of the user, the wearable apparatus 120 may hinder a body motion of the user or provide resistance to a body motion of the user by providing a resistance force to the body of the user. When the wearable apparatus 120 is, for example, a hip-type wearable apparatus, the wearable apparatus 120 may provide an exercise load to a body motion of the user while being worn on the legs, thereby enhancing the exercise effect of the user. The user may perform a walking motion while wearing the wearable apparatus 120 for exercise. In this case, the wearable apparatus 120 may apply a resistance force to the leg motion during the walking motion of the user.

In various embodiments, an example of a hip-type wearable apparatus 120 that is worn on the waist and legs is described for ease of description. However, as described above, the wearable apparatus 120 may be worn on another body part (e.g., the upper arms, lower arms, hands, calves, and feet) other than the waist and legs (particularly, the thighs), and the shape and configuration of the wearable apparatus 120 may vary depending on the body part on which the wearable apparatus 120 is worn.

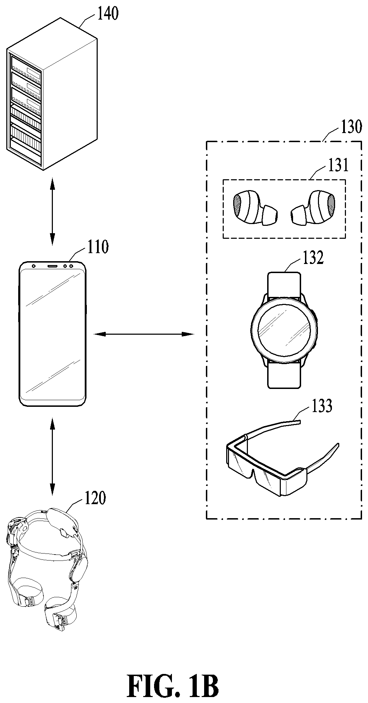

FIG. 1B is a diagram illustrating an example of a system including a wearable apparatus, according to an embodiment.

Referring to FIG. 1B, an electronic device 110 may communicate with the wearable apparatus 120 and remotely control the wearable apparatus 120. The electronic device 110 may be various types of devices. The electronic device 110 may include, for example, a portable communication device (e.g., a smartphone), a computer device, a portable multimedia device, or a home appliance but is not limited thereto.

According to an embodiment, the electronic device 110 and/or the wearable apparatus 120 may be connected to another wearable apparatus 130. For example, the wearable apparatus 120, the electronic device 110, and the other wearable apparatus 130 may be connected to each other through a wireless communication link (e.g., a Bluetooth communication link). The other wearable apparatus 130 may include, for example, wireless earphones 131, a smartwatch 132, or smart glasses 133, but is not limited thereto. The smartwatch 132 may be a watch-type wearable apparatus (or a watch-type electronic device), and the smart glasses 133 may be an eyewear-type wearable apparatus (or an eyewear-type electronic device).

In an embodiment, the smartwatch 132 may control the wearable apparatus 120. When the smartwatch 132 is connected to the electronic device 110 through a wireless communication link, and the electronic device 110 is connected to the wearable apparatus 120 through a wireless communication link, the smartwatch 132 may control the wearable apparatus 120 through the electronic device 110. However, embodiments are not limited thereto, and the smartwatch 132 may be directly connected to the wearable apparatus 120 and control the wearable apparatus 120.

In an embodiment, the electronic device 110 may transmit, to the other wearable apparatus 130, a control signal to instruct to provide a user with feedback corresponding to a state of the wearable apparatus 120. The other wearable apparatus 130 may provide (or output) feedback (e.g., at least one of visual feedback, auditory feedback, or haptic feedback) corresponding to the state of the wearable apparatus 120 in response to the reception of the control signal.

In an embodiment, the electronic device 110 may communicate with a server 140 using short-range wireless communication (e.g., wireless-fidelity (Wi-Fi)) or mobile communication (e.g., fourth generation (4G), 5G, etc.).

In an embodiment, the electronic device 110 may receive profile information of the user from the user. The profile information may include, for example, at least one of the age, gender, height, weight, or body mass index (BMI), or a combination thereof. The electronic device 110 may transmit the profile information of the user to the server 140.

In an embodiment, the electronic device 110 and/or the wearable apparatus 120 may request the user to perform one or more target motions to determine (or check) the exercise ability of the user. The one or more target motions may include, for example, a knee lift, a backward leg stretch, etc. The knee lift may be an exercise (or a motion) that starts from a position of the user standing straight with two feet on the ground and returns to the standing position after lifting a knee as much as possible without bending at the waist. The backward leg stretch may be an exercise (or a motion) that starts from a position of the user standing straight with the hands on the wall and returns to the standing position after lifting a leg backward as much as possible without bending at the waist.

In an embodiment, the wearable apparatus 120 may obtain motion information of the user performing a target motion using a sensor (e.g., an inertial measurement unit (IMU)) and may transmit the obtained motion information to the electronic device 110. The electronic device 110 may transmit the obtained motion information to the server 140.

In an embodiment, the server 140 may determine a target amount of exercise of the user for each of the exercise types (e.g., strength training, balance exercise, and aerobic exercise) through the profile information and motion information received from the electronic device 110. The server 140 may transmit the target amount of exercise for each of the exercise types to the electronic device 110.

In an embodiment, the server 140 may include a database in which information about a plurality of exercise programs to be provided to the user through the wearable apparatus 120 is stored. For example, the server 140 may manage a user account of the user of the electronic device 110 or the wearable apparatus 120. The server 140 may store and manage a workout program performed by the user and a result of performance with respect to the workout program in link with the user account.

In an embodiment, the electronic device 110 and/or the server 140 may provide the user with various exercise programs to achieve an exercise goal in various exercise environments desired by the user. The exercise goal may include, for example, at least one of muscle strength improvement, physical strength improvement, cardiovascular endurance improvement, core stability improvement, flexibility improvement, or symmetry improvement, or a combination thereof.

In an embodiment, the electronic device 110 and/or the server 140 may recommend exercise programs to the user to achieve the exercise goal of the user. Each exercise program may include one or more exercise modes. For example, each exercise mode may be about a body motion to achieve a predetermined exercise goal. For example, running may be an exercise mode for improving the cardiovascular endurance of the user. For example, a lunge may be an exercise mode for improving the core stability of the user. A combination of a plurality of exercise modes forming each exercise program may vary according to the exercise goal of the user. The electronic device 110 may provide the user with various exercise programs according to the combination of the plurality of exercise modes, even for the same exercise goal.

In an embodiment, the plurality of exercise modes may be stored in the electronic device 110 or the server 140 as a database. The electronic device 110 or the server 140 may generate the plurality of exercise programs based on a variety of information about the user and recommend a target exercise program among the plurality of exercise programs to the user considering the exercise goal or an exercise performance state of the user. For example, the electronic device 110 or the server 140 may determine the target exercise program to recommend to the user based on at least one of the exercise goal, an exercise history, or an exercise performance result of the user. Accordingly, a new exercise program may be recommended to the user even if the user performs an exercise every day under the same exercise goal, and the user may feel like performing a different exercise from the previous exercise by performing the new exercise program.

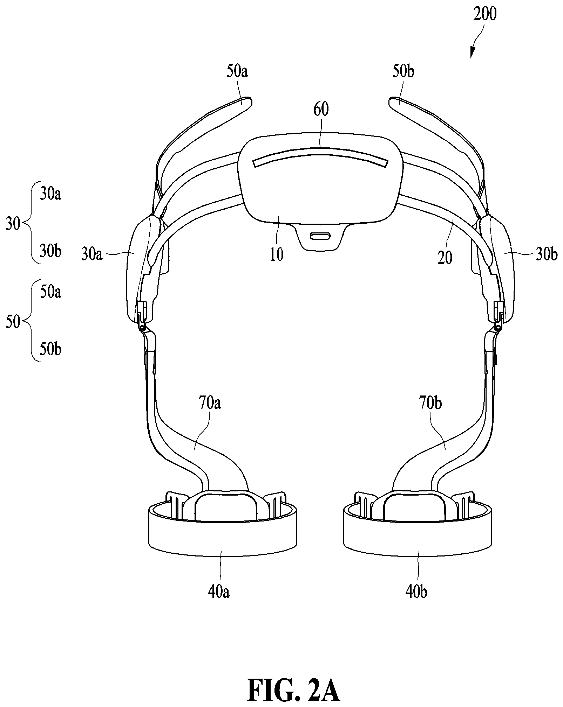

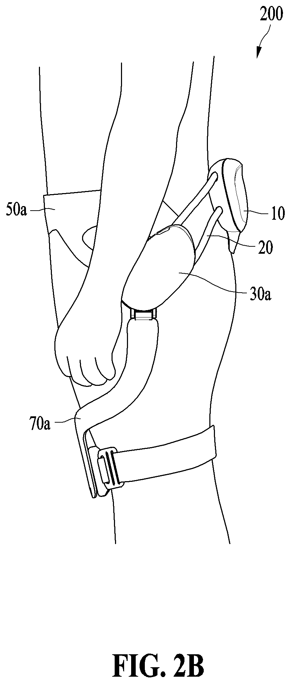

FIG. 2A is a rear schematic view of a wearable apparatus according to an embodiment. FIG. 2B is a left side view of a wearable apparatus according to an embodiment.

A wearable apparatus 200 shown in FIGS. 2A and 2B may be an example of the wearable apparatus 120.

Referring to FIG. 2A, the wearable apparatus 200 according to an embodiment may include a lumbar support module 10 comprising a lumbar support (or a first support) , a lumbar frame (or a second frame) 20, a driving module 30 comprising a motor and/or circuitry, thigh fastening portions 40a and 40b, a main belt 50, and thigh frames (or a first frame) 70a and 70b.

According to an embodiment, the lumbar support module 10 may be positioned on the lumbar region (lower back area) of the user while the user is wearing the wearable apparatus 200. The lumbar support module 10 may be mounted on the lumbar region of the user to provide a cushioning feeling to the waist of the user and support the waist of the user. The lumbar support module 10 may be hung on the hip region (an area of the hips) to prevent or reduce a chance of the wearable apparatus 200 from being downwardly separated due to gravity while the user is wearing the wearable apparatus 200. The lumbar support module 10 may distribute some of the weight of the wearable apparatus 200 to the waist of the user while the user is wearing the wearable apparatus 200. The lumbar support module 10 may be connected, directly or indirectly, to the lumbar frame 20. Connecting elements (not shown) that may be connected to the lumbar frame 20 may be formed at both end portions of the lumbar support module 10.

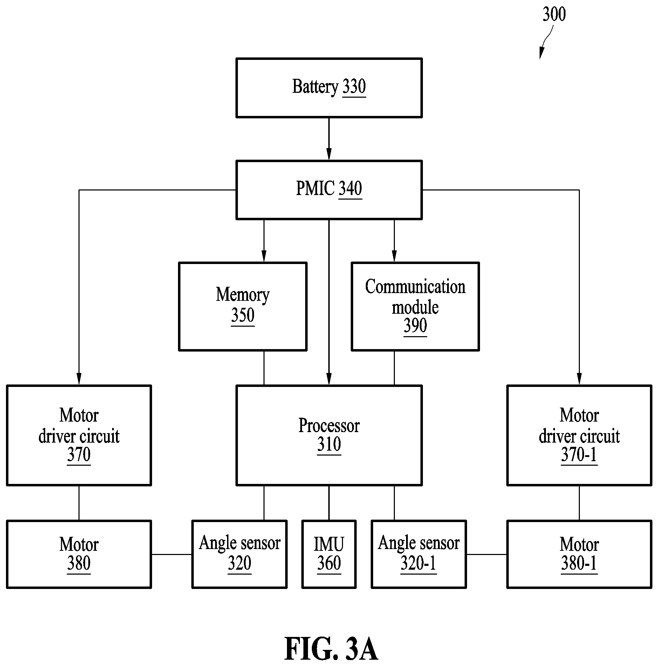

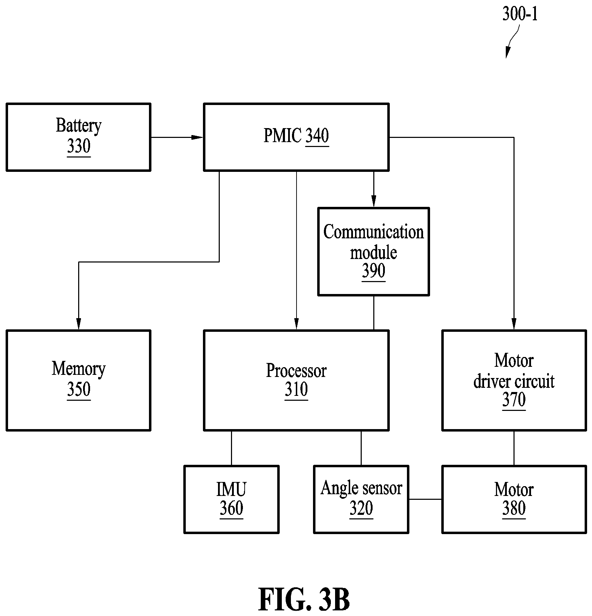

According to an embodiment, the lumbar support module 10 may include a lighting unit 60. The lighting unit 60 may include a plurality of light sources (e.g., light-emitting diodes (LEDs)). The lighting unit 60 may emit light by control of a processor (e.g., a processor 310 of FIGS. 3A and 3B described below). According to embodiments, the processor may control the lighting unit 60 such that visual feedback corresponding to the state of the wearable apparatus 200 (e.g., a booting state, a sensing state, etc.) may be provided (or output) to the user through the lighting unit 60. Each "processor" herein includes processing circuitry, and/or may include multiple processors. For example, as used herein, including the claims, the term "processor" may include various processing circuitry, including at least one processor, wherein one or more of at least one processor, individually and/or collectively in a distributed manner, may be configured to perform various functions described herein. As used herein, when "a processor", "at least one processor", and "one or more processors" are described as being configured to perform numerous functions, these terms cover situations, for example and without limitation, in which one processor performs some of recited functions and another processor(s) performs other of recited functions, and also situations in which a single processor may perform all recited functions. Additionally, the at least one processor may include a combination of processors performing various of the recited /disclosed functions, e.g., in a distributed manner. At least one processor may execute program instructions to achieve or perform various functions.

According to an embodiment, the lumbar support module 10 (e.g., the lumbar support) comprise a housing. The housing of the lumbar support module 10 (e.g., the lumbar support) may be disposed proximate to the lumbar region of the user and/or may support the lumbar region of the user when the wearable device 120 is worn on a body of the user.

According to an embodiment, the lumbar frame 20 may extend from both end portions of the lumbar support module 10. The lumbar region of the user may be accommodated inside the lumbar frame 20. The lumbar support frame 20 may include at least one rigid body beam. Each beam may be in a curved shape having a preset curvature to enclose the lumbar region of the user. The main belt 50 may be connected, directly or indirectly, to an end portion of the lumbar frame 20. The driving module 30 may be mounted on the lumbar frame 20. The lumbar frame 20 may include a connector (not shown) for mounting the driving module 30 thereon.

According to an embodiment, the driving module 30 may include a first driving module 30a positioned on the left side of the user while the user is wearing the wearable apparatus 200 and a second driving module 30b positioned on the right side of the user while the user is wearing the wearable apparatus 200.

According to an embodiment, the first driving module 30a may include a first angle sensor (e.g., a first encoder or a first Hall sensor) for measuring the angle (e.g., a left hip joint angle) of a first joint of the user. The second driving module 30b may include a second angle sensor (e.g., a second encoder or a second Hall sensor) for measuring the angle (e.g., a right hip joint angle) of a second joint of the user.

According to an embodiment, the first driving module 30a and the second driving module 30b may generate a torque. The first driving module 30a may be connected, directly or indirectly, to the first thigh frame 70a and the second driving module 30b may be connected, directly or indirectly, to the second thigh frame 70b. The first driving module 30a may provide the generated torque to the left leg of the user through the first thigh frame 70a. The first thigh frame 70a may provide an external force to the left leg of the user by rotating through the torque generated by the first driving module 30a. The second driving module 30b may provide the generated torque to the right leg of the user through the second thigh frame 70b. The second thigh frame 70b may provide an external force to the right leg of the user by rotating through the torque generated by the second driving module 30b.

According to an embodiment, the thigh frames 70a and 70b may support the legs (e.g., thighs) of the user when the wearable apparatus 200 is worn on the legs of the user. The thigh frames 70a and 70b may include the first thigh frame 70a for supporting the left leg of the user and the second thigh frame 70b for supporting the right leg of the user.

According to an embodiment, the thigh frames 70a and 70b may transmit a torque generated by, for example, the driving modules 30a and 30b to the thighs of the user. As the end portions of the thigh frames 70a and 70b may be respectively connected, directly or indirectly, to the driving modules 30a and 30b to rotate, and the other end portions of the thigh frames 70a and 70b may be respectively connected, directly or indirectly, to the thigh fastening portions 40a and 40b, the thigh frames 70a and 70b may support the thighs of the user and transmit the torque generated by the driving modules 30a and 30b to the thighs of the user. For example, the thigh frames 70a and 70b may push or pull the thighs of the user. The thigh frames 70a and 70b may extend in the longitudinal direction of the thighs of the user. The thigh frames 70a and 70b may be bent to surround at least a portion of the circumferences of the thighs of the user.

According to an embodiment, the thigh fastening portions 40a and 40b may be connected, directly or indirectly, to the thigh frames 70a and 70b and may fasten the thigh frames 70a and 70b to the thighs. The thigh fastening portions 40a and 40b may include a first thigh fastening portion 40a for fastening the first thigh frame 70a to the left thigh of the user and a second thigh fastening portion 40b for fastening the second thigh frame 70b to the right thigh of the user.

According to an embodiment, the first thigh fastening portion 40a may include a first cover, a first fastening frame, and a first strap, and the second thigh fastening portion 40b may include a second cover, a second fastening frame, and a second strap. The first cover and the second cover may be arranged on one of the sides of the thighs of the user. The first cover and the second cover may be arranged on the front surfaces of the thighs of the user. The first cover and the second cover may be arranged in the circumferential directions of the thighs of the user. The first cover and the second cover may extend to both sides from the other end portions of the thigh frames 70a and 70b and may include curved surfaces corresponding to the thighs of the user. Ends of the first cover and the second cover may be connected, directly or indirectly, to the fastening frames, and the other ends of the first cover and the second cover may be connected, directly or indirectly, to straps.

According to an embodiment, the first fastening frame and the second fastening frame may be arranged, for example, to surround at least some portions of the circumferences of the thighs of the user, thereby preventing or reducing chances of the thighs of the user from being separated from the thigh frames 70a and 70b. The first fastening frame may have a fastening structure that connects the first cover to a first strap, and the second fastening frame may have a fastening structure that connects the second cover to a second strap.

According to an embodiment, the first strap may enclose the remaining portion of the circumference of the left thigh of the user that is not covered by the first cover and the first fastening frame, and the second strap may enclose the remaining portion of the circumference of the right thigh of the user that is not covered by the second cover and the second fastening frame. The first strap and the second strap may include, for example, an elastic material (e.g., a band).

According to an embodiment, the main belt 50 may be connected, directly or indirectly, to the lumbar frame (or the second frame) 20. The main belt 50 may include a first main belt 50a configured to enclose the left abdomen of the user while the user is wearing the wearable apparatus 200, and a second main belt 50b configured to enclose the right abdomen of the user while the user is wearing the wearable apparatus 200. The first main belt 50a may be formed in a shape having a longer length than the second main belt 50b but is not limited thereto, and the first main belt 50a may be formed in a shape having the same length as or a shorter length than the second main belt 50b. The first main belt 50a and the second main belt 50b may be connected to both end portions of the second frame 20, respectively. The main belt 50 may be bent in a direction to surround the abdomen of the user when the body of the user is inserted in such a direction that the body is accommodated in the wearable apparatus 200. The first main belt 50a and the second main belt 50b may be connected to each other while the user is wearing the wearable apparatus 200. The main belt 50 may distribute a portion of the weight of the wearable apparatus 200 to the abdomen of the user while the user is wearing the wearable apparatus 200.

Referring to FIG. 2B, the lumbar support module 10 may be mounted on the back of the lumbar region of the user and be hung on the hip region of the user, thereby supporting some of the weight of the wearable apparatus 200. The first driving module 30a may be arranged on the left lumbar region of the user. The second frame 20 may extend from an end portion of the lumbar support module 10 and be inclined in a direction toward the first driving module 30a. The first main belt 50a mounted on the second frame 20 may surround the left abdomen of the user.

FIGS. 3A and 3B are block diagrams illustrating examples of a configuration of a wearable apparatus, according to an embodiment.

According to an embodiment, a wearable apparatus 300 of FIG. 3A may include a processor 310, angle sensors 320 and 320-1, a battery 330, a power management integrated circuit (PMIC) 340, a memory 350, an IMU(or IMU sensor) 360, motor driver circuits 370 and 370-1, motors (or actuators) 380 and 380-1, and a communication module 390.

Although the plurality of angle sensors 320 and 320-1, the plurality of motor driver circuits 370 and 370-1, and the plurality of motors 380 and 380-1 are shown in FIG. 3, which is merely an example, a wearable apparatus 300-1 in the example shown in FIG. 3B may include a single angle sensor 320, a single motor driver circuit 370, and a single motor 380. Also, depending on the implementation, the wearable apparatuses 300 and 300-1 may include a plurality of processors. The number of motor driver circuits, the number of motors, or the number of processors may vary depending on a body part on which the wearable apparatuses 300 and 300-1 are worn.

The wearable apparatus 300 of FIG. 3A and the wearable apparatus 300-1 of FIG. 3B may be examples of the wearable apparatus 120 and the wearable apparatus 200.

According to an embodiment, the angle sensor 320, the motor driver circuit 370, and the motor 380 may be included in the first driving module 30a of FIG. 2A, and the angle sensor 320-1, the motor driver circuit 370-1, and the motor 380-1 may be included in the second driving module 30b of FIG. 2A. Each "driving module" herein may include a motor and/or a driving circuit, and may optionally include an angle sensor.

According to an embodiment, the angle sensor 320 and the angle sensor 320-1 may each correspond to a Hall sensor but are not limited thereto.

According to an embodiment, the angle sensor 320 may measure or sense the angle of the first thigh frame 70a (or the angle of the first joint (e.g., the left hip joint, etc.) of the user). The angle sensor 320 may transmit the measurement result (e.g., an angle value of the angle of the first thigh frame 70a) to the processor 310.

According to an embodiment, the angle sensor 320-1 may measure or sense the angle of the second thigh frame 70b (or the angle of the second joint (e.g., the right hip joint) of the user). The angle sensor 320 may transmit the measurement result (e.g., an angle value of the angle of the second thigh frame 70b) to the processor 310.

According to an embodiment, the angle sensor 320 and the angle sensor 320-1 may additionally measure the knee angles and ankle angles of the user according to the positions of the angle sensor 320 and the angle sensor 320-1.

According to an embodiment, the wearable apparatuses 300 and 300-1 may include a potentiometer. The potentiometer may sense an R-axis joint angle, an L-axis joint angle, an R-axis joint angular velocity, and an L-axis joint angular velocity according to a walking motion of the user. The R/L axis may be reference axes for the right leg and the left leg of the user, respectively. For example, the R/L axis may be set to be vertical to the ground and set such that the front side of the body of a person has a negative value and the rear side of the body has a positive value.

According to an embodiment, the PMIC 340 may charge the battery 330 using power supplied from an external power source. For example, the external power source may be connected to the wearable apparatuses 300 and 300-1 through a cable (e.g., a universal serial bus (USB) cable, etc.). The PMIC 340 may receive power from the external power source through the cable and charge the battery 330 using the received power. According to embodiments, the PMIC 340 may charge the battery 330 through a wireless charging method.

According to an embodiment, the PMIC 340 may transmit power stored in the battery 330 to a component (e.g., the processor 310, the memory 350, the IMU 360, the communication module 390, etc.) in the wearable apparatuses 300 and 300-1. The PMIC 340 may, for example, adjust the power stored in the battery 330 to a voltage or current level suitable for the components in the wearable apparatus 300. The PMIC 340 may include, for example, a converter (e.g., a direct current (DC)-DC converter) or a regulator (e.g., a low-dropout (LDO) regulator or a switching regulator) configured to perform the adjustment described above.

According to an embodiment, the PMIC 340 may determine state information (e.g., a state of charge, a state of health, an overvoltage, a low voltage, an overcurrent, an overcharge, an overdischarge, an overheating, a short circuit, or a swelling) of the battery 330 and transmit the state information of the battery 330 to the processor 310. The processor 310 may provide the state information of the battery 330 to the user. For example, the processor 310 may output the status information of the battery 330 through at least one of a sound output module (e.g., a speaker), a vibration output module (e.g., a vibration motor or a haptic motor), or a display module (e.g., a display or the lighting unit 60). For example, the processor 310 may transmit the state information of the battery 330 to the electronic device 110 through the communication module 390, and the electronic device 110 may display the state information of the battery 330 on the display.

According to an embodiment, the IMU 360 may obtain motion information of the wearable apparatuses 300 and 300-1 (or the user). For example, the IMU 360 may obtain rotation angle values (e.g., an angle value of an X rotation angle, an angle value of a Y rotation angle, and an angle value of a Z rotation angle) of the lumbar support module 10 (or the user). The X rotation angle may be, for example, an angle at which the lumbar support module 10 rotates about the X-axis, the Y rotation angle may be, for example, an angle at which the lumbar support module 10 rotates around the Y-axis, and the Z rotation angle may be, for example, an angle at which the lumbar support module 10 rotates around the Z-axis. The IMU 360 may transmit the obtained motion information (e.g., rotation angle values) to the processor 310. Depending on the implementation, the IMU 360 may, for example, obtain three-axis (e.g., X-axis, Y-axis, Z-axis) acceleration values and angular acceleration values of the lumbar support module 10 (or the user) and may transmit the obtained acceleration values and angular acceleration values to the processor 310. The processor 310 may determine rotation angle values of the lumbar support module 10 (or the user) based on at least some of the obtained acceleration values and the angular acceleration values.

According to an embodiment, the processor 310 may control the overall operation of the wearable apparatuses 300 and 300-1.

According to an embodiment, the processor 310 may be operatively connected to at least one or all of the angle sensors 320 and 320-1, the memory 350, or the IMU 360.

According to an embodiment, the processor 310 may, for example, control the components (e.g., the motor driver circuits 370 and 370-1, etc.) in the wearable apparatuses 300 and 300-1 by executing software (e.g., a program or instructions) stored in the memory 350, and perform various data processing or computations. As at least a portion of the data processing or computations, the processor 310 may store data received from other components (e.g., the IMU 360, the angle sensors 320 and 320-1, etc.) in the memory 350 and process the instructions or data stored in the memory 350.

According to an embodiment, the motor driver circuits 370 and 370-1 may control the motors 380 and 380-1, respectively, under the control by the processor 310, and each of the motors 380 and 380-1 may generate a torque by this control.

According to an embodiment, the communication module 390 may support the establishment of a direct (e.g., wired) communication channel or a wireless communication channel between the wearable apparatus 300, 300-1 and an external electronic device, and support the communication through the established communication channel. The communication module may include one or more communication processors configured to support direct (e.g., wired) communication or wireless communication. According to an embodiment, the communication module may include a wireless communication module (e.g., a cellular communication module, a short-range wireless communication module, or a global navigation satellite system (GNSS) communication module) or a wired communication module (e.g., a local area network (LAN) communication module or a power line communication (PLC) module). A corresponding one of these communication modules may communicate with the external electronic device via a first network (e.g., a short-range communication network such as Bluetooth^{™}, Wi-Fi direct, or infrared data association (IrDA)) or a second network (e.g., a legacy cellular network, a 5G network, a next-generation communication network, the Internet, or a computer network). These various types of communication modules may be implemented as a single component (e.g., a single chip), or may be implemented as multiple components (e.g., multiple chips) separate from each other.

According to an embodiment, the wearable apparatuses 300 and 300-1 may include a display module. The display module may include, for example, a display and/or a lighting unit (e.g., the lighting unit 60 of FIG. 2A). The processor 310 may control the display module so that the display module may provide visual feedback to the user.

According to an embodiment, the wearable apparatuses 300 and 300-1 may include a sound output module. The sound output module may include, for example, one or more speakers. The processor 310 may control the sound output module so that the sound output module may provide auditory feedback to the user.

According to an embodiment, the wearable apparatuses 300 and 300-1 may include a vibration output module. The vibration output module may include, for example, one or more vibration motors or one or more haptic motors. The processor 310 may control the vibration output module so that the vibration output module may provide tactile feedback (or haptic feedback) to the user.

According to an embodiment, at least one of the processor 310, the battery 330, the PMIC 340, the memory 350, the IMU 360, the communication module 390, the display module, the sound output module, or the vibration output module, or a combination thereof may be positioned in the lumbar support module 10 of FIGS. 2A and 2B.

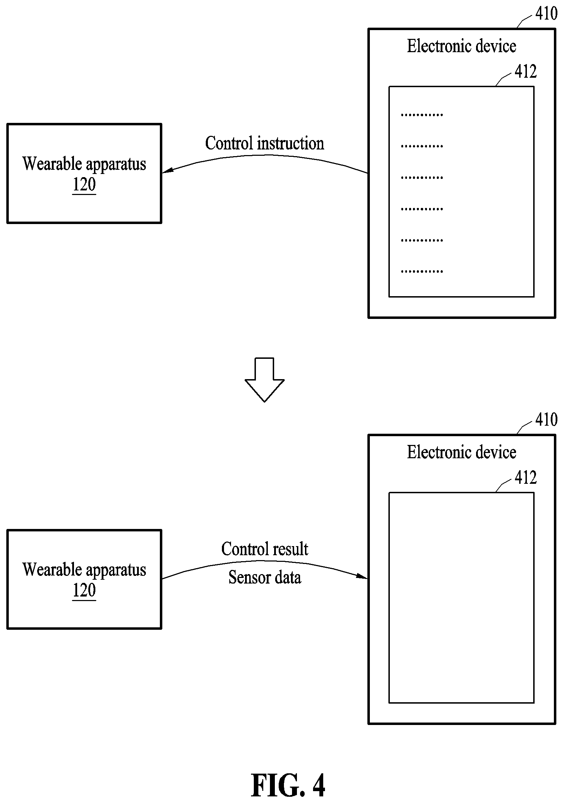

FIG. 4 is a diagram illustrating an interaction between a wearable apparatus and an electronic device, according to an embodiment.

Referring to FIG. 4, the wearable apparatus 120 may communicate with an electronic device 410 (e.g., a smartphone or smartwatch). For example, the electronic device 410 may be a user terminal of the user who uses the wearable apparatus 120 or a controller device dedicated to the wearable apparatus 120. According to an embodiment, the wearable apparatus 120 and the electronic device 410 may be connected to each other through short-range wireless communication (e.g., Bluetooth^{™} or Wi-Fi communication).

According to an embodiment, the electronic device 410 may verify a state of the wearable apparatus 120 or execute an application to control or operate the wearable apparatus 120. A screen of a user interface (UI) may be displayed to control an operation of the wearable apparatus 120 or determine an operation mode of the wearable apparatus 120 on a display 412 of the electronic device 410 through the execution of the application. The UI may be, for example, a graphical UI (GUI).

According to an embodiment, the user may input an instruction to control an operation of the wearable apparatus 120 through the GUI screen on the display 412 of the electronic device 410 (e.g., an instruction to instruct the wearable apparatus 120 to operate in an assistance mode of generating an assistance force or an instruction to instruct the wearable apparatus 120 to operate in a resistance mode of generating a resistance force) or change settings of the wearable apparatus 120. The electronic device 410 may generate a control instruction (or control signal) corresponding to an operation control instruction or a setting change instruction input by the user and transmit the generated control instruction to the wearable apparatus 120. The wearable apparatus 120 may operate according to the received control instruction and transmit a control result according to the control instruction and/or sensor data measured by the sensor (e.g., the angle sensors 320 and 320-1 and/or the IMU 360) of the wearable apparatus 120 to the electronic device 410. The electronic device 410 may provide the user with result information (e.g., walking ability information, exercise ability information, or exercise posture evaluation information) derived by analyzing the control result and/or the sensor data through the GUI screen.

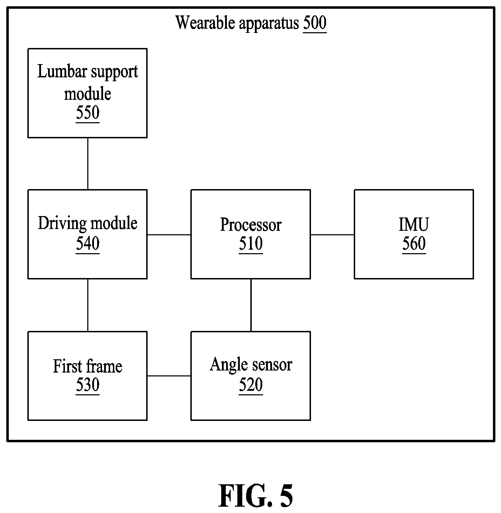

FIG. 5 is a block diagram illustrating an example of a configuration of a wearable apparatus, according to an embodiment.

Referring to FIG. 5, a wearable apparatus 500 (e.g., the wearable apparatus 120, the wearable apparatus 200, the wearable apparatus 300, and the wearable apparatus 300-1) according to an embodiment may include a processor 510 (e.g., the processor 310), an angle sensor 520, a first frame 530, a driving module 540 (e.g., the driving module 30), a lumbar support module 550 (e.g., the lumbar support module 10), and an IMU 560 (or an IMU sensor) (e.g., the IMU 360).

According to an embodiment, the first frame 530 may correspond to a portion of the lower body (e.g., the thighs) of the user. The first frame 530 may include, for example, the first thigh frame 70a and/or the second thigh frame 70b.

According to an embodiment, the angle sensor 520 may include the angle sensor 320 and/or the angle sensor 320-1. The angle sensor 520 may obtain a first frame angle value (e.g., a first thigh frame angle value and/or a second thigh frame angle value) by sensing the angle of the first frame 530. The angle sensor 520 may transmit the obtained first frame angle value to the processor 510. The processor 510 may obtain the first frame angle value using the angle sensor 520. For example, the angle sensor 320 may obtain a first thigh frame angle value by sensing the angle of the first thigh frame 70a and transmit the obtained first thigh frame angle value to the processor 510. The angle sensor 320-1 may obtain a second thigh frame angle value by sensing the angle of the second thigh frame 70b and transmit the obtained second thigh frame angle value to the processor 510. The processor 510 may obtain the first thigh frame angle value using the angle sensor 320 and obtain the second thigh frame angle value using the angle sensor 320-1.

According to an embodiment, the driving module 540 may include one or more motors (e.g., the motor 380 and/or the motor 380-1) and one or more motor driver circuits (e.g., the motor driver circuit 370 and/or the motor driver circuit 370-1). The driving module 540 may include the first driving module 30a and/or the second driving module 30b.

According to an embodiment, the lumbar support module 550 may be connected to the driving module 540 and may be on the lower back area of the user.

According to an embodiment, the IMU 560 may obtain motion values of the lumbar support module 550. The motion values may include, for example, rotation angle values (e.g., an angle value of the X rotation angle, an angle value of the Y rotation angle, and an angle value of the Z rotation angle) of the lumbar support module 550.

According to an embodiment, the processor 510 may receive the motion values of the lumbar support module 550 from the IMU 560. The processor 510 may obtain the motion values of the lumbar support module 550 using the IMU 560. The processor 510 may evaluate a sitting posture of the user based on at least some (e.g., the angle value of the Y rotation angle) of the motion values of the lumbar support module 550 and the first frame angle value received from the angle sensor 520. The processor 510 may determine whether the sitting posture of the user is an abnormal sitting posture based on at least some of the motion values received from the IMU 560 and the first frame angle value received from the angle sensor 520.

According to an embodiment, when determining that the sitting posture of the user is the abnormal sitting posture, the processor 510 may control the driving module 540 such that the driving module 540 generates a first torque. For example, the first torque may be a torque in a first rotation direction (e.g., clockwise). The lumbar support module 550 may perform a rotational motion based on the first torque generated by the driving module 540, and through this rotational motion, the lumbar support module 550 may push the lower back area of the user. As the lumbar support module 550 provides an external force (e.g., force pushing the lower back area) to the lower back area of the user, the pelvis and spine of the user may rotate so that the abnormal sitting posture of the user may be corrected by the wearable apparatus 500. The wearable apparatus 500 may provide the user with an external force to correct the abnormal sitting posture of the user.

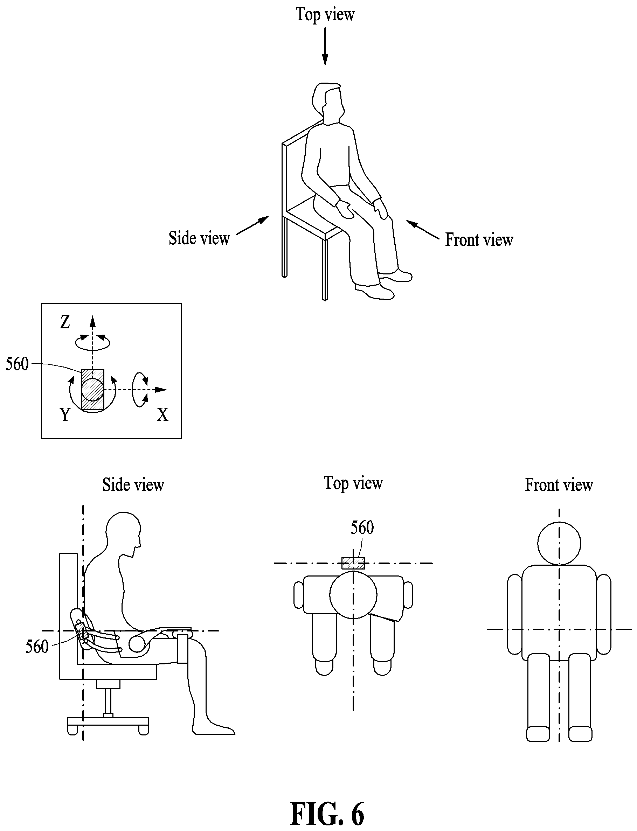

Hereinafter, operations of the wearable apparatus 500 are described in detail with reference to FIGS. 6 to 16.

FIG. 6 is a diagram illustrating an example of a rotation angle of a wearable apparatus, according to an embodiment.

Referring to FIG. 6, the side view may correspond to the Z-axis direction, the top view may correspond to the Y-axis direction (or gravity direction), and the front view may correspond to the X-axis direction.

According to an embodiment, the IMU 560 may be located within the lumbar support module 550 and may obtain motion values of the lumbar support module 550 (or the IMU 560).

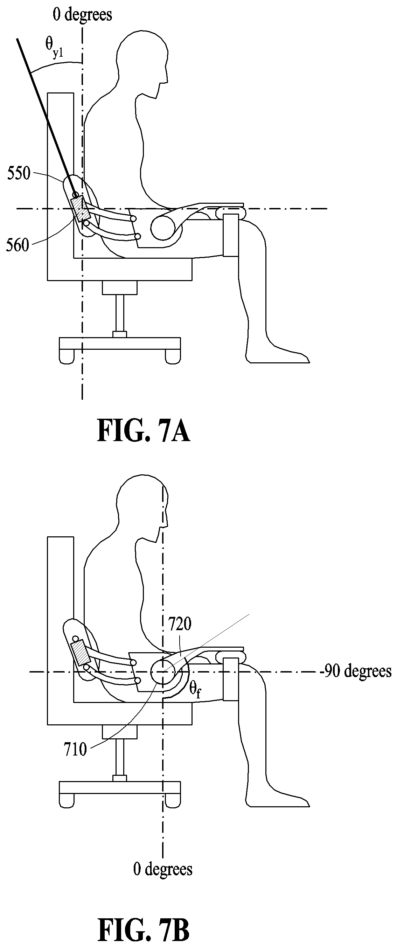

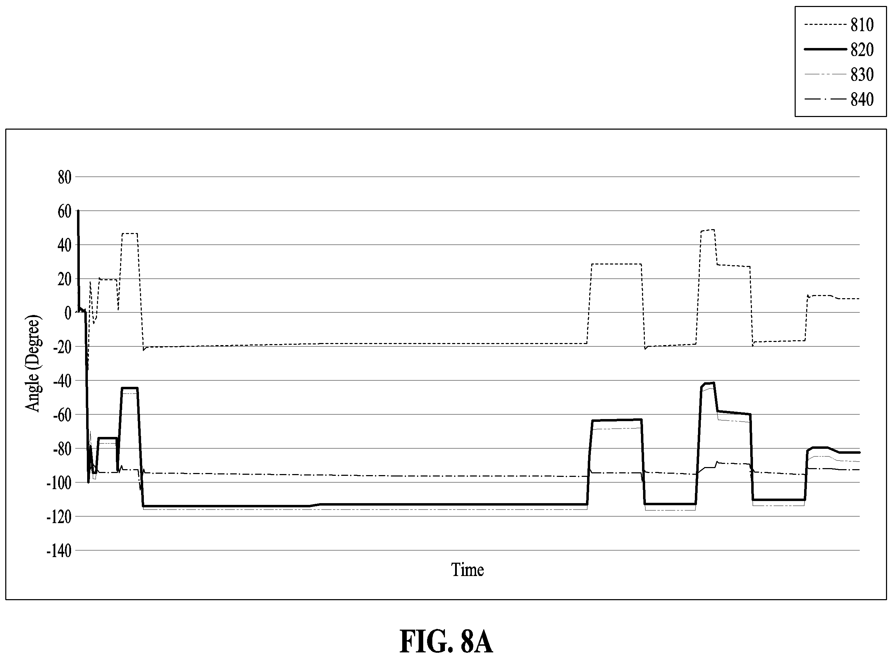

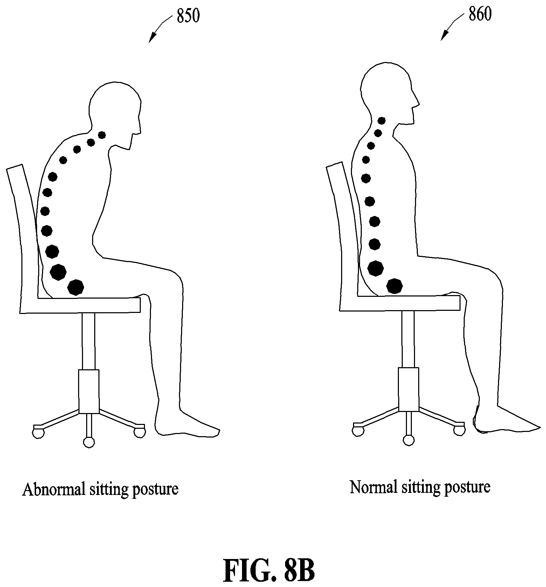

According to an embodiment, the processor 510 may obtain the motion values (e.g., rotation angle values) of the lumbar support module 550 (or the IMU 560) using the IMU 560. The processor 510 may receive the first frame angle value from the angle sensor 520. The processor 510 may obtain the first frame angle value using the angle sensor 520. As described in detail with reference to FIGS. 7A, 7B, 8A, and 8B, the processor 510 may evaluate the sitting posture of the user based on at least some of the motion values of the lumbar support module 550 and the first frame angle value.

FIGS. 7A, 7B, 8A, and 8B are diagrams illustrating examples of an operation in which a wearable apparatus evaluates a sitting posture of a user, according to an embodiment.

In the example shown in FIG. 7A, the lumbar support module 550 may rotate about a first axis (e.g., Y-axis), and this rotation may cause the lumbar support module 550 to form an angle with the first axis (e.g., Y-axis). The angle formed by the lumbar support module 550 with the first axis may correspond to the "Y rotation angle" described above. The "Y rotation angle" may be expressed as pitch or an Euler Y angle. Although not shown in FIG. 7A, the lumbar support module 550 may form an angle (e.g., "X rotation angle") with a second axis (e.g., X-axis) and/or form an angle (e.g., "Z rotation angle") with a third axis (e.g., Z-axis). The "X rotation angle" may be expressed as roll or an Euler X angle, and the "Z rotation angle" may be expressed as yaw or an Euler Z angle.

In the example shown in FIG. 7A, the IMU 560 may transmit the motion values (e.g., first motion values) of the lumbar support module 550 to the processor 510. A first rotation angle value θ_{y1} of FIG. 7A may be included in the first motion values. Depending on the implementation, the processor 510 may obtain the acceleration value in the X-axis direction, the acceleration value in the Y-axis direction, and the acceleration value in the Z-axis direction of the lumbar support module 550 using the IMU 560 and calculate the first rotation angle value θ_{y1} using at least one or all of the obtained acceleration values.

In the example shown in FIG. 7B, the angle sensor 520 may obtain the first frame angle value (e.g., first and second thigh frame angle values) by sensing the angle of the first frame 530. For example, the angle sensor 520 (e.g., the angle sensor 320-1 of FIG. 3A) of a driving module 710 (e.g., the second driving module 30b) may obtain a second thigh frame angle value by sensing the angle of a second thigh frame 720 (e.g., the second thigh frame 70b). The second thigh frame angle value may be the angle value between the Y-axis marked 0 degrees in FIG. 7B and the second thigh frame 70b. Although not shown in FIG. 7B, the angle sensor 520 (e.g., the angle sensor 320 of FIG. 3A) may obtain a first thigh frame angle value by sensing the angle of the first thigh frame (e.g., the first thigh frame 70a). The first thigh frame angle value may be the angle value between the Y-axis marked 0 degrees in FIG. 7B and the first thigh frame 70a.

FIG. 8A illustrates a graph 810 of Y rotation angle over time, a graph 820 of the angle of the second thigh frame over time, and a graph 830 of the angle of the first thigh frame over time.

According to an embodiment, the processor 510 may determine or calculate the angle value θ_{f} (or the angle at which the first frame 530 is separated from the Y-axis) between the Y-axis and the first frame 530 (e.g., the first and second thigh frames 70a and 70b) by subtracting the Y rotation angle (e.g., the first rotation angle value θ_{y1}) from the average of the second thigh frame angle (e.g., the second thigh frame angle value) and the first thigh frame angle (e.g., the first thigh frame angle value). A graph 840 of FIG. 8A may be a graph for the angle value θ_{f}. When the upper body of the user moves, the Y rotation angle may change. Accordingly, the angle of the second thigh frame and the angle of the first thigh frame may change as shown in the graphs 820 and 830. Due to the movement of the upper body of the user, the first rotation angle value θ_{y1} may be included as noise in the second thigh frame angle value and the first thigh frame angle value. The processor 510 may calculate the average value of the second thigh frame angle value and the first thigh frame angle value and determine the angle value θ_{f} between the Y-axis and the first frame 530 by subtracting the first rotation angle value θ_{y1} from the calculated average value. In other words, the processor 510 may correct the average value of the second thigh frame angle value and the first thigh frame angle value by subtracting the first rotation angle value θ_{y} from the average value of the second thigh frame angle value and the first thigh frame angle value. The angle value θ_{f} may correspond to the corrected average value.

According to an embodiment, the processor 510 may determine whether the user is in a sitting posture based on the angle value θ_{f}. When the user is in a sitting posture, the angle value θ_{f} may have a value around -90 degrees, as shown in the graph 840. The processor 510 may determine that the user is not in a sitting posture when the angle value θ_{f} is not around -90 degrees (e.g., when the angle value θ_{f} does not fall within a second angle range). The processor 510 may determine that the user is in a sitting posture when the angle value θ_{f} is around -90 degrees (e.g., when the angle value θ_{f} falls within the second angle range). The second angle range may be, for example, -85 degrees to -95 degrees, but is not limited thereto.

According to an embodiment, when determining that the user is in a sitting posture, the processor 510 may determine whether the first rotation angle value θ_{y1} falls within the first angle range (or normal range) (e.g., 0 to 20 degrees). When determining that the first rotation angle value θ_{y1} does not fall within the first angle range (or normal range), the processor 510 may determine that the sitting posture of the user is an abnormal sitting posture. When determining that the first rotation angle value θ_{y1} falls within the first angle range (or normal range), the processor 510 may determine that the sitting posture of the user is a normal sitting posture.

According to an embodiment, the processor 510 may estimate or determine the angle at which the lumbar vertebrae of the user rotates around the X-axis using the angle value of the X rotation angle of the lumbar support module 550. The processor 510 may determine whether the lumbar vertebrae of the user forms rightward tilting or leftward tilting using the angle value of the X rotation angle of the lumbar support module 550. For example, the rightward tilting may be, for example, the angle at which the lumbar vertebrae of the user is tilted to the right from the X-axis, while the leftward tilting may be, for example, the angle at which the lumbar vertebrae of the user is tilted to the left from the X-axis. The processor 510 may determine that the lumbar vertebrae of the user is not in a normal lumbar vertebrae shape when the angle value of the X rotation angle is greater than or equal to a predetermined level.

According to an embodiment, the processor 510 may estimate or determine the angle at which the lumber vertebrae of the user rotates around the Z-axis using the angle value of the Z rotation angle of the lumbar support module 550. The processor 510 may determine that the lumbar vertebrae of the user is not in a normal lumbar vertebrae shape when the angle value of the Z rotation angle is greater than or equal to a predetermined level.

FIG. 8B illustrates an example of the abnormal sitting posture and an example of the normal sitting posture.

In the example illustrated in FIG. 8B, an abnormal sitting posture 850 may include, for example, a posture in which the lumbar vertebrae of the user is bent backward to form a kyphotic curve when the user is sitting. A normal sitting posture 860 may include, for example, a posture in which the lumbar vertebrae of the user is bent forward to form a lordotic curve and a posture in which the thoracic vertebrae of the user forms a kyphotic curve when the user is sitting.

According to an embodiment, the processor 510 may transmit, to an electronic device, at least one or all of the angle value θ_{f}, the first rotation angle value, the angle value of the X rotation angle, or the angle value of the Z rotation angle via the communication module 390. The electronic device may display a screen (or GUI) indicating that the user is in a normal sitting posture or an abnormal sitting posture based on the angle value and the first rotation angle value received from the wearable apparatus 500. The electronic device may display the angle at which the lumber vertebrae of the user rotates around the X-axis and/or Z-axis.

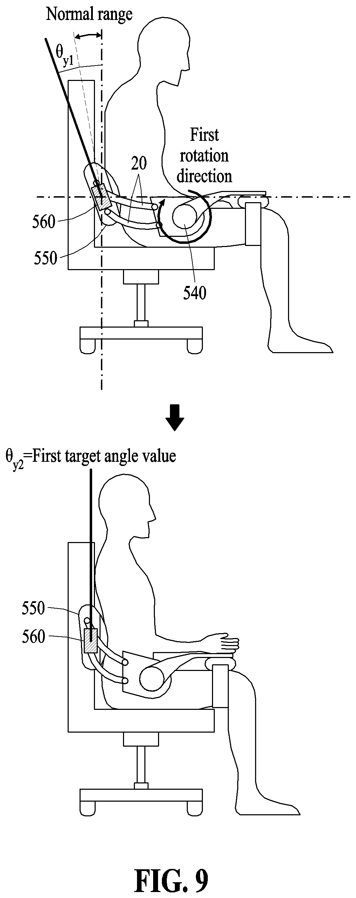

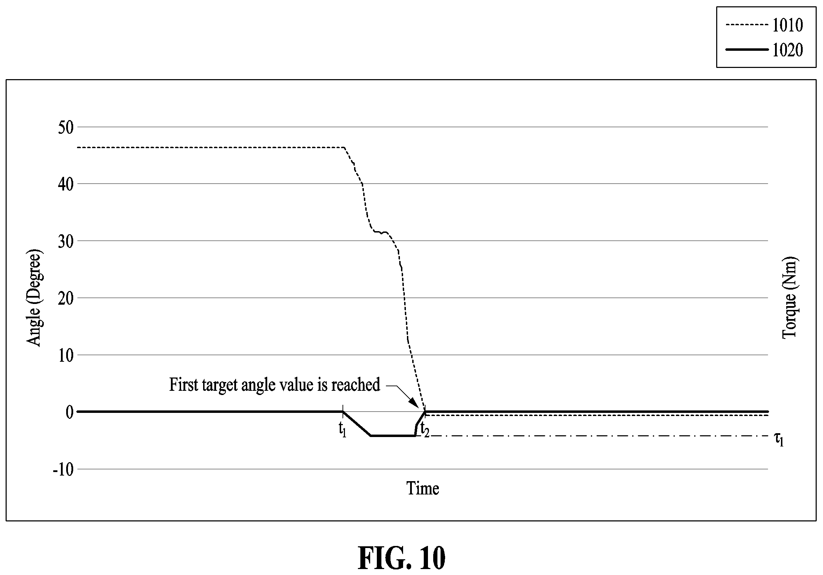

FIGS. 9 and 10 are diagrams illustrating examples of an operation in which a wearable apparatus rotates a lumbar support module by generating a first torque, according to an embodiment.

As in the example shown in FIG. 9, the processor 510 may determine that the first rotation angle value θ_{y1} is outside the first angle range (or normal range). In this case, the processor 510 may determine that the sitting posture of the user is an abnormal sitting posture (e.g., the abnormal sitting posture 850 of FIG. 8B).

According to an embodiment, when determining that the sitting posture of the user is the abnormal sitting posture, the processor 510 may control the driving module 540 such that the driving module 540 (e.g., the first driving module 30a and/or the second driving module 30b) generates a first torque. For example, when determining that the sitting posture of the user is the abnormal sitting posture, the processor 510 may control the motor driver circuit 370 and/or the motor driver circuit 370-1 such that the motor 380 and/or the motor 380-1 generates the first torque.

According to an embodiment, the processor 510 may determine the torque value (hereinafter, "first torque value") of the first torque and control the driving module 540 such that the first torque having a magnitude corresponding to the first torque value may be generated by the driving module 540. For example, the processor 510 may determine the first torque value based on the angle value (e.g., the first rotation angle value θ_{y1}) of the Y rotation angle of the lumbar support module 550. The larger the angle value of the Y rotation angle, the larger the first torque value may be determined. In another example, the electronic device may receive, from the user, a magnitude value (or intensity value) used to determine a torque magnitude. The electronic device may transmit the received magnitude value to the wearable apparatus 500. The processor 510 may determine the first torque value based on the magnitude value received from the electronic device. The larger the magnitude value, the larger the first torque value may be determined. In another example, the processor 510 may determine the first torque value using the angle value (e.g., the first rotation angle value θ_{y1}) of the Y rotation angle and the received magnitude value. In another example, the processor 510 may determine the first torque value using the angle value of the Y rotation angle, the received magnitude value, and profile information (e.g., age and gender) of the user.

According to an embodiment, the lumbar support module 550 may provide an external force to the user by performing a rotational motion based on the first torque generated by the driving module 540. This external force may correspond to a force pushing the lower back area of the user. For example, the lumbar support module 550 may be connected to the driving module 540 through the lumbar frame 20. The first torque generated by the driving module 540 may rotate the lumbar frame 20, and the lumbar support module 550 may perform a rotational motion according to the rotation of the lumbar frame 20. A force pushing the lower back area of the user may be provided to the user through the rotation of the lumbar support module 550.

According to an embodiment, the wearable apparatus 500 may generate the first torque and then increase the magnitude of the first torque up to a target magnitude value (e.g., a first target magnitude value to be described below). The wearable apparatus 500 may reduce the magnitude of the first torque when the Y rotation angle of the lumbar support module 550 reaches the target angle value (e.g., a first target angle value to be described below).

According to an embodiment, the IMU 560 may also rotate according to the rotational motion of the lumbar support module 550. The IMU 560 may obtain motion values (hereinafter, "second motion values") of the lumbar support module 550 (or the IMU 560) that performs the rotational motion based on the first torque. The second motion values may include, for example, rotation angle values of the lumbar support module 550 (or the IMU 560) that performs the rotational motion based on the first torque.

According to an embodiment, the processor 510 may receive the second motion values of the lumbar support module 550 from the IMU 560. The processor 510 may obtain the second motion values of the lumbar support module 550 using the IMU 560. The second rotation angle value θ_{y2} of FIG. 9 may be included in the second motion values. For example, the second rotation angle value θ_{y2} may correspond to the angle value at which the lumbar support module 550, which performs the rotational motion based on the first torque, rotates around the first axis (e.g., Y axis).

According to an embodiment, the processor 510 may determine whether the second rotation angle value θ_{y2} corresponds to (or reaches) the first target angle value (e.g., 0 degrees). When determining that the second rotation angle value θ_{y2} corresponds to the first target angle value (or the second rotation angle value θ_{y2} reaches the first target angle value), the processor 510 may perform an operation of preventing or reducing chances of the driving module 540 from generating the first torque or an operation of controlling the driving module 540 such that the driving module 540 generates a torque with an intensity less than the intensity of the first torque.

FIG. 10 illustrates an example of a graph 1010 of the Y rotation angle of the lumbar support module 550 and an example of a graph 1020 of the first torque when the lumbar support module 550 performs a rotational motion based on the first torque.

In the example shown in FIG. 10, the wearable apparatus 500 may generate the first torque through the driving module 540 at a time point t₁. From the time point t₁, the magnitude of the first torque may increase. When the magnitude of the first torque reaches a first target magnitude value τ₁ (or falls within a first target magnitude range based on the first target magnitude value τ₁), the magnitude of the first torque may have the first target magnitude value τ₁ for a predetermined period of time. For a predetermined period of time, the magnitude of the first torque may be maintained at the first target magnitude value τ₁.

According to an embodiment, the processor 510 may determine the first target magnitude value τ₁ based on, for example, the first rotation angle value θ_{y1}. The larger the first rotation angle value θ_{y1}, the larger the first target magnitude value τ₁ may be. The processor 510 may determine the first target magnitude value τ₁ through the degree (e.g., the difference value between the first rotation angle value and 20 degrees) to which the first rotation angle value θ_{y1} deviates from the first angle range (e.g., 20 to 0 degrees). In another example, the electronic device may receive, from a user, a target value (or magnitude value) used to determine the target torque magnitude. The electronic device may transmit the received target value (or magnitude value) to the wearable apparatus 500. The processor 510 may determine the first target magnitude value based on the target value (or magnitude value) received from the electronic device. In another example, the processor 510 may determine the first target magnitude value using the first rotation angle value θ_{y1}, the received target value, and profile information (e.g., age, gender, etc.) of the user In another example, the first target magnitude value may be predetermined.

According to an embodiment, while the first torque is generated, the second frame 20 may be rotated by the generated first torque, and the lumbar support module 550 connected to the second frame 20 may perform a rotational motion by the rotation (e.g., the rotation in the first rotation direction) of the second frame 20. By this rotational motion (e.g., the rotational motion in the first rotation direction), the lumbar support module 550, comprising a lumbar support, may provide an external force to a portion of the body part (e.g., the lower back area) of the user (or push a portion of the body part of the user). When the sitting posture of the user is an abnormal sitting posture, the wearable apparatus 500 may provide the external force to the lower back area of the user such that the lumbar vertebrae of the user forms a lordotic curve.

According to an embodiment, the wearable apparatus 500 may provide the external force to the user, so the Y rotation angle of the lumbar support module 550 after the time point t₁ may decrease. At a time point t₂, the processor 510 may obtain second motion values of the lumbar support module 550 using the IMU 560.

According to an embodiment, the processor 510 may determine whether the second rotation angle value θ_{y2} among the obtained second motion values corresponds to the first target angle value.

According to an embodiment, when determining that the second rotation angle value θ_{y2} corresponds to the first target angle value, the processor 510 may operate such that the wearable apparatus 500 does not provide an external force (e.g., an external force generated by the rotational motion of the lumbar support module 550) to the user. For example, the processor 510 may control the driving module 540 such that the driving module 540 does not generate (or output) the first torque. The processor 510 may control the motor driver circuits 370 and 370-1 such that current does not flow in the motors 380 and 380-1.

According to an embodiment, when determining that the second rotation angle value θ_{y2} corresponds to the first target angle value, the processor 510 may operate such that the wearable apparatus 500 provides a smaller external force to the user. For example, the processor 510 may control the driving module 540 such that the first torque having a magnitude (e.g., τ₁/n) less than a magnitude (e.g., the first target magnitude value τ₁) is generated. n may be, for example, 5 but is not limited thereto. When the second rotation angle value θ_{y2} corresponds to the first target angle value, the driving module 540 may generate the first torque having the magnitude τ_{1/n}.

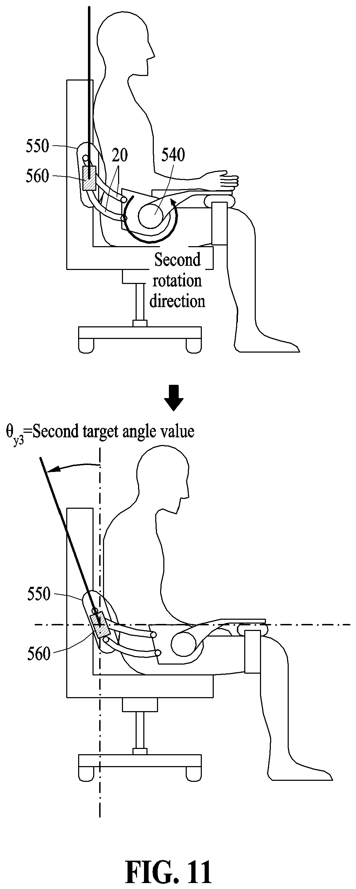

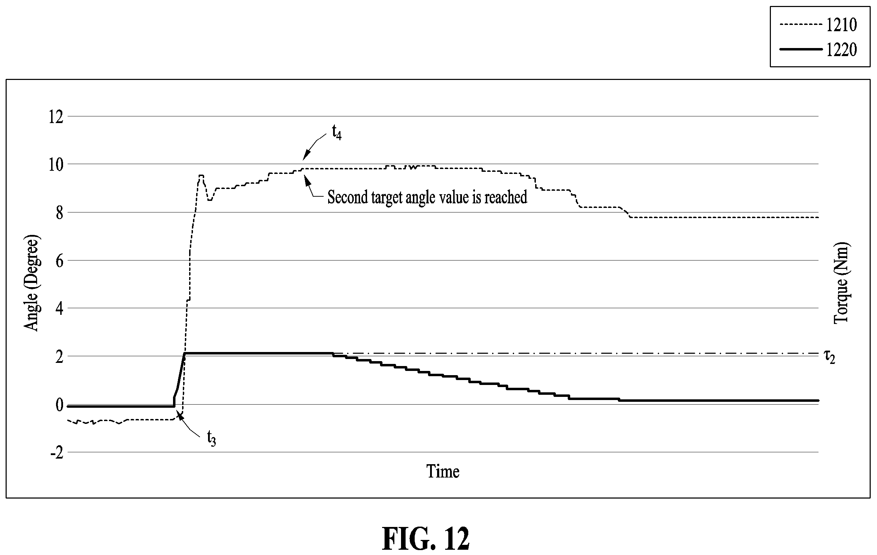

FIGS. 11 and 12 are diagrams illustrating examples of an operation in which a wearable apparatus rotates the lumbar support module by generating a second torque, according to an embodiment.

In the example shown in FIG. 11, according to an embodiment, the processor 510 may check whether the rotation angle values of the lumbar support module 550 obtained using the IMU 560 after the second rotation angle value θ_{y2} maintain a state corresponding to the first target angle value (e.g., 0 degrees) during a first time. The first time may be, for example, two minutes but is not limited thereto. The first time may be adjusted.

According to an embodiment, when the rotation angle values of the lumbar support module 550 obtained after the second rotation angle value maintain the state corresponding to the first target angle value during the first time, the processor 510 may control the driving module 540 such that the driving module 540 generates a second torque. For example, the second torque may be a torque in a second rotation direction (e.g., counterclockwise).

According to an embodiment, the lumbar support module 550 may perform a rotational motion based on the second torque generated by the driving module 540. The lumbar support module 550 may be connected to the driving module 540 through the lumbar frame 20. The second torque generated by the driving module 540 may rotate the lumbar frame 20, and the lumbar support module 550 may perform a rotational motion in the second rotation direction according to the rotation of the lumbar frame 20. A force applied to the lower back area of the user may decrease according to the rotational motion of the lumbar support module 550 in the second rotation direction.

According to an embodiment, the IMU 560 may also rotate according to the rotational motion of the lumbar support module 550 in the second rotation direction. The IMU 560 may obtain motion values (hereinafter, "third motion values") of the lumbar support module 550 (or the IMU 560) that performs the rotational motion based on the second torque. The third motion values may include, for example, the rotation angle values of the lumbar support module 550 (or the IMU 560) that performs the rotational motion based on the second torque.

According to an embodiment, the processor 510 may obtain the third motion values of the lumbar support module 550 using the IMU 560. A third rotation angle value θ_{y3} of FIG. 11 may be included in the third motion values. For example, the third rotation angle value θ_{y3} may correspond to the angle value at which the lumbar support module 550, which performs a rotational motion based on the second torque, rotates around the first axis (e.g., Y axis).

According to an embodiment, the processor 510 may determine whether the third rotation angle value θ_{y3} corresponds to (or reaches) the second target angle value (e.g., 10 degrees). When determining that the third rotation angle value θ_{y3} corresponds to the second target angle value (or the third rotation angle value θ_{y3} reaches the second target angle value), the processor 510 may perform an operation of preventing or reducing chances of the driving module 540 from generating the second torque or an operation of controlling the driving module 540 such that the driving module 540 generates a torque with an intensity smaller than the intensity of the second torque.

FIG. 12 illustrates an example of a graph 1210 of the Y rotation angle of the lumbar support module 550 and an example of a graph 1220 of the second torque when the lumbar support module 550 performs a rotational motion based on the second torque.

In the example shown in FIG. 12, the wearable apparatus 500 may generate the first torque having a magnitude (e.g., τ_{1/n}) or may not generate the first torque before a time point t₃.

The wearable apparatus 500 may generate the second torque through the driving module 540 at the time point t₃. From the time point t₃, the magnitude of the second torque may increase. When the magnitude of the second torque falls within the second target magnitude range (or reaches the second target magnitude value τ₂), the magnitude of the second torque may have the second target magnitude value τ₂ during a predetermined period of time. For a predetermined period of time, the magnitude of the second torque may be maintained at the second target magnitude value τ₂.

According to an embodiment, while the second torque is generated, the second frame 20 may be rotated by the generated second torque, and the lumbar support module 550 connected to the second frame 20 may perform a rotational motion by the rotation (e.g., the rotation in the second rotation direction) of the second frame 20. Due to this rotational motion (e.g., rotational motion in the second rotation direction), the lumbar support module 550 may move away from a portion of the body part (e.g., the lower back area) of the user.

According to an embodiment, the Y rotation angle of the lumbar support module 550 may increase after the time point t₃ according to the rotational motion of the lumbar support module 550 in the second rotation direction. At a time point t₄, the processor 510 may receive third motion values of the lumbar support module 550 from the IMU 560. The processor 510 may determine whether the third rotation angle value θ_{y3} among the third motion values of the lumbar support module 550 corresponds to the second target angle value (e.g., 10 degrees).

According to an embodiment, when determining that the third rotation angle value θ_{y3} corresponds to the second target angle value, the wearable apparatus 500 may not generate the second torque. For example, the processor 510 may control the driving module 540 such that the driving module 540 does not generate (or output) the second torque. The processor 510 may control the motor driver circuits 370 and 370-1 such that current does not flow in the motors 380 and 380-1.

According to an embodiment, when determining that the third rotation angle value θ_{y3} corresponds to the second target angle value, the processor 510 may operate such that the driving module 540 generates the second torque of less magnitude. For example, when determining that the third rotation angle value θ_{y3} corresponds to the second target angle value, the processor 510 may control the driving module 540 such that the second torque having a magnitude (e.g., τ₂/n) less than a magnitude (e.g., the second target magnitude value τ₂) is generated. n may be, for example, 5 but is not limited thereto. When the third rotation angle value θ_{y3} corresponds to the second target angle value, the driving module 540 may generate the second torque having the magnitude τ_{2/n}.

According to an embodiment, the operations of the wearable apparatus 500 described with reference to FIGS. 9 and 10 and the operations of the wearable apparatus 500 described with reference to FIGS. 11 and 12 may be included in a mode (hereinafter, "first mode" (or posture correction mode or posture balancing mode)) in which the wearable apparatus 500 assists a user in achieving a sitting posture closer to a normal sitting posture (or enables the user to maintain a normal sitting posture). In the first mode, when determining that the sitting posture of the user is an abnormal sitting posture, the wearable apparatus 500 may output the first torque. When the angle value of the Y rotation angle of the lumbar support module 550 that performs a rotational motion based on the first torque reaches the first target angle value (e.g., 0 degrees), the wearable apparatus 500 may not output the first torque or may output a torque (e.g., a torque with an intensity sufficient to maintain the angle value of the Y rotation angle at the first target angle value) less than the intensity of the first torque. After a predetermined period of time elapses, the wearable apparatus 500 may output the second torque. When the angle value of the Y rotation angle of the lumbar support module 550 that performs a rotational motion based on the second torque reaches the second target angle value (e.g., about 10 degrees), the wearable apparatus 500 may not output the second torque or may output a torque (e.g., τ_{2/n}) less than the intensity of the second torque.

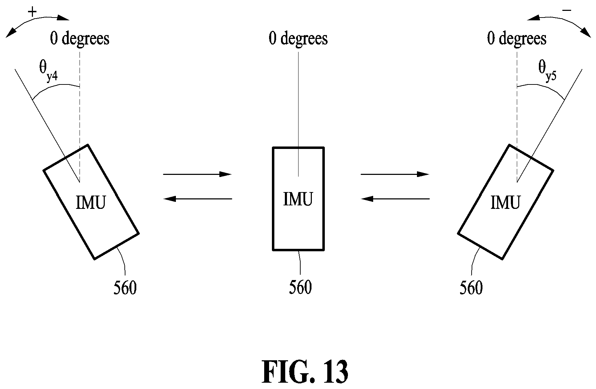

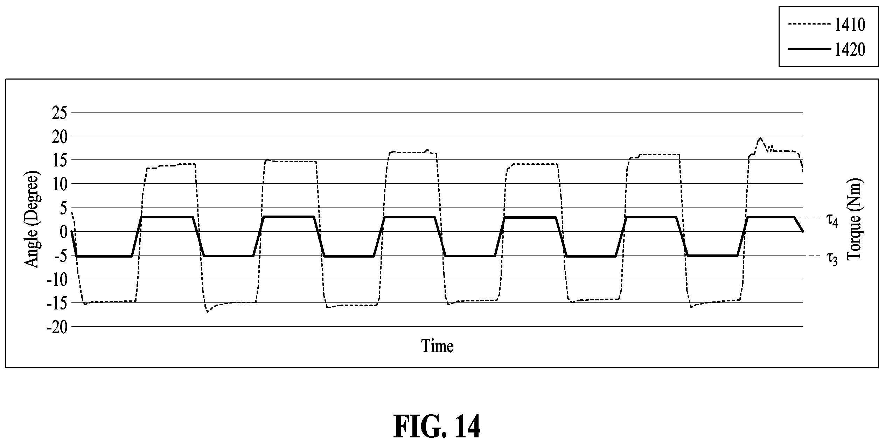

In an embodiment to be described below, the wearable apparatus 500 may operate in a mode (hereinafter "second mode (or repetitive exercise mode)") that may strengthen the muscle strength (e.g., lower back muscle strength) of the user in a sitting posture by repeatedly outputting the first torque and the second torque. This will be described with reference to FIGS. 13 and 14.

FIGS. 13 and 14 are diagrams illustrating examples of a wearable apparatus operating in a mode in which the wearable apparatus may strengthen the muscles of a user in a sitting posture, according to an embodiment.

In the example shown in FIG. 13, when the Y rotation angle of the lumbar support module 550 (or the IMU 560) is behind the 0-degree position of the first axis (e.g., Y axis), the Y rotation angle may be positive, and when the Y rotation angle of the lumbar support module 550 (or the IMU 560) is in front of the 0-degree position of the first axis (e.g., Y axis), the Y rotation angle may be negative.

According to an embodiment, the wearable apparatus 500 may determine that the angle value of the Y rotation angle of the lumbar support module 550 is a fourth rotation angle value θ_{y4} when the user is in a sitting posture. In a state in which the angle value of the Y rotation angle has the fourth rotation angle value θ_{y4}, the wearable apparatus 500 may output the first torque. As described with reference to FIGS. 9 and 10, the lumbar support module 550 may perform a rotational motion driven by the first torque. As the lumbar support module 550 performs the rotational motion driven by the first torque, the angle value of the Y rotation angle may have a fifth rotation angle value θ_{y5}, as shown in the example shown in FIG. 13. In a state in which the angle value of the Y rotation angle has the fifth rotation angle value θ_{y5}, the wearable apparatus 500 may output a second torque.

According to an embodiment, as described with reference to FIGS. 11 and 12, the lumbar support module 550 may perform a rotational motion driven by the second torque. As the lumbar support module 550 performs the rotational motion driven by the second torque, the angle value of the Y rotation angle may have the fourth rotation angle value θ_{y4}, as shown in the example shown in FIG. 13. In a state in which the angle value of the Y rotation angle has the fourth rotation angle value θ_{y4}, the wearable apparatus 500 may output the first torque.

According to an embodiment, the wearable apparatus 500 may repeatedly output the first torque and the second torque in a second mode (that is, a mode in which the muscles of the user in a sitting posture may be strengthened). FIG. 14 illustrates examples of a graph 1410 of the Y rotation angle and a torque graph 1420 of the lumbar support module 550 when the wearable apparatus 500 operates in the second mode.

In the example shown in FIG. 14, the wearable apparatus 500 may repeatedly output the first torque and the second torque. In the torque graph 1420, an interval, in which a torque value is a negative number, may be an interval in which the first torque is output, and an interval, in which the torque value is a positive number, may be an interval in which the second torque is output.

In the example shown in FIG. 14, when the wearable apparatus 500 outputs the first torque, the lumbar support module 550 may rotate in the first rotation direction. As the lumbar support module 550 rotates in the first rotation direction, the lumbar support module 550 may rotate forward and the Y rotation angle of the lumbar support module 550 may have a negative number. When the magnitude value of the first torque is, for example, the magnitude value τ₃ (e.g., about -5 newton-meters (Nm)) of FIG. 14, the angle value of the Y rotation angle may be the fifth rotation angle value (e.g., about -15 degrees).

According to an embodiment, the wearable apparatus 500 may increase the magnitude (or intensity) of the first torque until the magnitude of the first torque reaches the magnitude value τ₃. When the magnitude of the first torque reaches the magnitude value τ₃, the wearable apparatus 500 may maintain the magnitude of the first torque at the magnitude value τ₃ for a predetermined period of time. While the magnitude of the first torque is maintained at the magnitude value τ₃, the angle value of the Y rotation angle of the lumbar support module 550 may be maintained at the fifth rotation angle value (e.g., about -15 degrees). Depending on the implementation, the wearable apparatus 500 may increase the intensity of the first torque until the angle value of the Y rotation angle of the lumbar support module 550 has the fifth rotation angle value (e.g., about -15 degrees).

After a predetermined period of time elapses, the wearable apparatus 500 may reduce the magnitude of the first torque and output the second torque by changing the rotation direction of the torque. When the wearable apparatus 500 outputs the second torque, the lumbar support module 550 may rotate in the second rotation direction. As the lumbar support module 550 rotates in the second rotation direction, the angle value of the Y rotation angle may approach 0 degrees and may have a positive number. When the magnitude value of the second torque is, for example, the magnitude value τ₄ (e.g., about 3 Nm) of FIG. 14, the angle value of the Y rotation angle may be the fourth rotation angle value (e.g., about 15 degrees).

According to an embodiment, the wearable apparatus 500 may increase the magnitude of the second torque until the magnitude of the second torque reaches the magnitude value τ₄. When the magnitude of the second torque reaches the magnitude value τ₄, the wearable apparatus 500 may maintain the intensity of the second torque at the magnitude value τ₄ for a predetermined period of time. While the magnitude of the second torque is maintained at the magnitude value τ₄, the angle value of the Y rotation angle of the lumbar support module 550 may be maintained at the fourth rotation angle value (e.g., about 15 degrees). Depending on the implementation, the wearable apparatus 500 may increase the intensity of the second torque until the angle value of the Y rotation angle of the lumbar support module 550 has the fourth rotation angle value (e.g., about 15 degrees).

According to an embodiment, in the second mode, the wearable apparatus 500 may repeatedly (or alternately) output the first torque and the second torque, as shown in the torque graph 1420.

According to an embodiment, the processor 510 of the wearable apparatus 500 may determine the angle value (e.g., the first rotation angle value described above) of the Y rotation angle of the lumbar support module 550 to evaluate the sitting posture of a user and determine (or evaluate) that the sitting posture of the user is an abnormal sitting posture through the determined first rotation angle value.

According to an embodiment, based on a degree (e.g., the difference value between the first rotation value and 20 degrees) to which the determined first rotation angle value deviates from the first angle range (e.g., 20 to 0 degrees), the processor 510 may determine a time interval value (hereinafter, referred to as "first interval value") during which the first torque is output (or the first torque is sustained) and a target magnitude value (hereinafter, referred to as "third target magnitude value") of the first torque and determine a time interval value (hereinafter, referred to as "second interval value") during which the second torque is output (or the second torque is sustained) and a target magnitude value (hereinafter, referred to as "fourth target magnitude value") of the second torque. The third target magnitude value may be, for example, the magnitude value τ₃ of FIG. 14, but is not limited thereto. The fourth target magnitude value may be, for example, the magnitude value τ₄ of FIG. 14 but is not limited thereto. Depending on the implementation, an electronic device may receive, from the user, a magnitude value and a time interval value related to the magnitude of a target torque and transmit the received magnitude value and time interval value to the wearable apparatus 500. The processor 510 may determine the third target magnitude value and the fourth target magnitude value based on the magnitude value received from the electronic device and determine the first interval value and the second interval value based on the time interval value received from the electronic device.

According to an embodiment, when it is determined that the wearable apparatus 500 operates in the second mode (e.g., when it is determined that the wearable apparatus 500 operates in the second mode based on user input from among the first mode and the second mode), the processor 510 may generate an exercise program (or determine a torque pattern) based on the third target magnitude value, the fourth target magnitude value, the first interval value, and the second interval value. In the second mode, the processor 510 may control the driving module 540 such that a torque is output according to the generated exercise program (or determined torque pattern). The torque output according to the generated exercise program (or determined torque pattern) may have the form of the torque graph 1420 of FIG. 14.

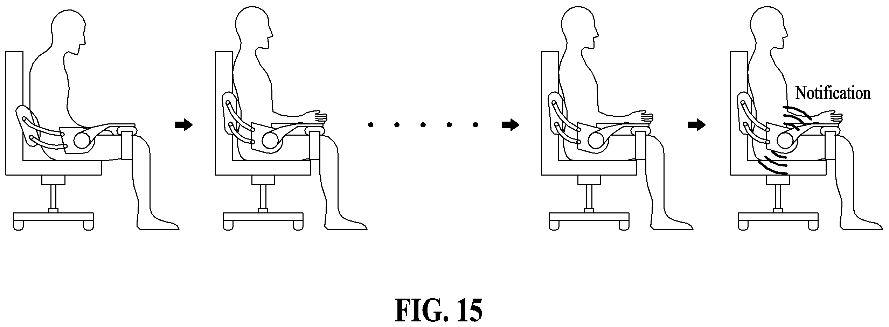

FIG. 15 is a diagram illustrating an example of an operation in which a wearable apparatus provides a notification, according to an embodiment.

In the example shown in FIG. 15, the wearable apparatus 500 may operate in the first mode or the second mode.

According to an embodiment, the wearable apparatus 500 (e.g., the processor 510) may measure the execution time of a mode (e.g., the first mode or the second mode) and determine whether the measured execution time exceeds a second time (e.g., one hour). The wearable apparatus 500 (e.g., the processor 510) may determine whether the operation time of the mode (e.g., the first mode or second mode) elapses the second time (e.g., one hour).

According to an embodiment, when the measured execution time exceeds the second time (or when the operation time of the mode elapses the second time), the wearable apparatus 500 (e.g., the processor 510) may provide a notification to the user.

For example, in the first mode or the second mode, the lumbar support module 550 may perform a rotational motion driven by the first torque (or second torque) generated by the driving module 540. When the measured execution time exceeds the second time, the processor 510 may control the driving module 540 (e.g., the first and second driving modules 30a and 30b) such that the first torque (or second torque) contributing to the rotational motion of the lumbar support module 550 is not generated. The processor 510 may control the driving module 540 such that the driving module 540 rotates the first frame 530 (e.g., the first and second thigh frames 70a and 70b). Under the control by the processor 510, the driving module 540 may generate a torque such that the first frame 530 may perform a rotational motion in the first rotation direction. As the first frame 530 performs the rotational motion in the first rotation direction (e.g., clockwise), an external force may be applied to a thigh of the user. Under the control by the processor 510, the driving module 540 may generate a torque such that the first frame 530 may perform a rotational motion in the second rotation direction (e.g., counterclockwise). As the first frame 530 performs the rotational motion in the second rotation direction, the external force may not be applied to the thigh of the user. The rotational motion in the first rotation direction and the rotational motion in the second rotation direction of the first frame 530 may be repeated, thereby repeatedly applying and releasing external force to the thighs of the user. This repetition may correspond to a notification similar to haptic feedback.

Depending on the implementation, when the measured execution time exceeds the second time (or when the operation time of a mode elapses the second time), the wearable apparatus 500 (e.g., the processor 510) may provide a voice notification to the user.

According to an embodiment, the wearable apparatus 500 (e.g., the processor 510) may provide a notification to the user such that the user may take a rest and/or stretch.

According to an embodiment, the wearable apparatus 500 (e.g., the processor 510) may detect whether the posture of the user is changed from a sitting posture to a standing posture after providing the notification. For example, the processor 510 may detect whether the posture of the user is changed from the sitting posture to the standing posture through an acceleration value (e.g., an acceleration value in the direction of gravity) obtained by the IMU 560.

According to an embodiment, when the user is in the standing posture, the wearable apparatus 500 (e.g., the processor 510) may request the electronic device to display a posture (or exercise) for the stretching of the user on a display of the electronic device. The wearable apparatus 500 (e.g., the processor 510) may generate a torque and provide the torque to the user such that the user may perform a posture (or exercise) displayed on the display of the electronic device.

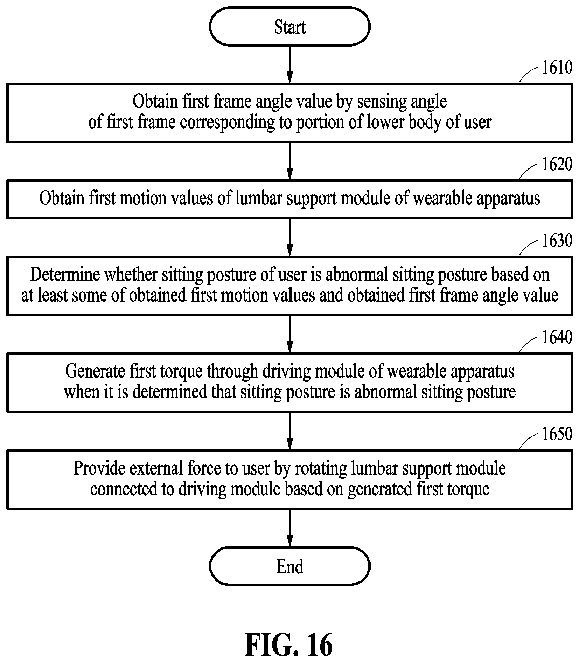

FIG. 16 is a flowchart illustrating an example of a method of operating a wearable apparatus, according to an embodiment.

Referring to FIG. 16, in operation 1610, the wearable apparatus 500 may obtain a first frame angle value by sensing the angle of the first frame 530 corresponding to a portion of the lower body of a user.

In operation 1620, the wearable apparatus 500 may obtain first motion values of the lumbar support module 550 of the wearable apparatus 500.

In operation 1630, the wearable apparatus 500 may determine whether the sitting posture of the user is an abnormal sitting posture based on at least some of the obtained first motion values and the obtained first frame angle value.

In operation 1640, when determining that the sitting posture of the user is the abnormal sitting posture, the wearable apparatus 500 may generate a first torque through the driving module 540 of the wearable apparatus 500.

In operation 1650, the wearable apparatus 500 may provide an external force to the user by rotating the lumbar support module 550 connected to the driving module 540 based on the generated first torque.

The embodiments described with reference to FIGS. 1 to 15 may apply to the method of operating the wearable apparatus 500.

According to an embodiment, a wearable apparatus 120, 200, 300, 300-1, 500 may include a driving module 540, a first frame 530 corresponding to a portion of the lower body of a user, an angle sensor 520 obtaining a first frame angle value by sensing the angle of the first frame, a lumbar support module 550 connected to the driving module and positioned on the lower back area of the user, an IMU 560, and a processor 510. The processor may obtain the first frame angle value using the angle sensor. The processor may obtain a first rotation angle value of the lumbar support module using the IMU. The processor may determine whether the sitting posture of the user is an abnormal sitting posture based on the first rotation angle value and the first frame angle value. When determining that the sitting posture is the abnormal sitting posture, the processor may control the driving module such that the driving module generates a first torque.

The lumbar support module may provide an external force to the user by performing a rotational motion based on the generated first torque.

The processor may determine whether the posture of the user corresponds to the sitting posture using the first frame angle value and the first rotation angle value, determine whether the first rotation angle value falls within a first angle range when determining that the posture of the user corresponds to the sitting posture, and determine that the sitting posture is the abnormal sitting posture when determining that the first rotation angle value is outside the first angle range.

When determining that the first rotation angle value is outside the first angle range, the processor may control the driving module such that the driving module generates the first torque in a first rotation direction.

The lumbar support module may provide an external force pushing the lower back area by performing a rotational motion in the first rotation direction based on the first torque.

In a state in which the first torque is generated, the processor may determine a second rotation angle value of the lumbar support module using the IMU, determine whether the second rotation angle value corresponds to a first target angle value, and when determining that the second rotation angle value corresponds to the first target angle value, perform an operation of preventing the driving module from generating the first torque or an operation of controlling the driving module such that the driving module generates a torque with an intensity less than the intensity of the generated first torque.

When rotation angle values of the lumbar support module, determined after the second rotation angle value, maintain a state corresponding to the first target angle value during a first time, the processor may control the driving module such that the driving module generates a second torque in a second rotation direction.

In a state in which the second torque is generated, the processor may determine a third rotation angle value of the lumbar support module using the IMU, determine whether the third rotation angle value corresponds to a second target angle value, and when determining that the third rotation angle value corresponds to the second target angle value, perform an operation of preventing the driving module from generating the second torque or an operation of controlling the driving module such that the driving module generates a torque with an intensity less than the intensity of the second torque.

The first frame may include a first thigh frame corresponding to a first thigh of the user and a second thigh frame corresponding to a second thigh of the user.

The processor may calculate an average value of a first angle value (e.g., a first thigh frame angle value), which is obtained by sensing the angle of the first thigh frame, and a second angle value (e.g., a second thigh frame angle value), which is obtained by sensing the angle of the second thigh frame, determine a third angle value (e.g., an angle value θ_{f}) by subtracting the first rotation angle value from the calculated average value, determine whether the determined third angle value falls within a second angle range, and determine that the posture of the user corresponds to the sitting posture when the determined third angle value falls within the second angle range.

The wearable apparatus may further include a second frame 20 connecting the driving module to the lumbar support module and rotated by the generated first torque.

As the second frame is rotated by the generated first torque, the lumbar support module may perform the rotational motion.

The processor may determine a magnitude value of the first torque based on at least one of the first rotation angle value and a magnitude value related to a torque magnitude received from an electronic device wirelessly connected to the wearable apparatus and control the driving module such that the first torque of the determined magnitude value is generated.

The processor may determine whether the execution time of a first mode that provides the external force to the user exceeds a second time and when determining that the execution time elapses the second time, control the wearable apparatus such that a notification is provided to the user.

When determining that the execution time elapses the second time, the processor may control the driving module such that the first frame connected to the driving module repeats a rotational motion in the first rotation direction and a rotational motion in the second rotation direction.

The first frame may repeatedly provide an external force corresponding to the notification to a portion of the lower body by repeating the rotational motion in the first rotation direction and the rotational motion in the second rotation direction.

The processor may operate in a second mode in which the first torque and the second torque are generated alternately.

In the second mode, the lumbar support module may alternately perform the rotational motion in the first rotation direction based on the generated first torque and the rotational motion in the second rotation direction based on the second torque.

The processor may determine at least one magnitude value of the first torque and the second torque and determine a time interval value for a duration of at least one of the first torque and the second torque, based on the first rotation angle value, and control the driving module such that the first torque and the second torque are generated alternately based on the determined magnitude value and the determined time interval value.

According to an embodiment, a method of operating a wearable apparatus 120, 200, 300, 300-1, 500 may include obtaining a first frame angle value by sensing the angle of a first frame 530 corresponding to a portion of the lower body of a user, obtaining a first rotation angle value of a lumbar support module 550 of the wearable apparatus, determining whether a sitting posture of the user is an abnormal sitting posture based on the first rotation angle value and the first frame angle value, generating a first torque through a driving module 540 of the wearable apparatus when determining that the sitting posture is the abnormal sitting posture, and providing an external force to the user by rotating the lumbar support module connected to the driving module based on the generated first torque.

The determining of whether the sitting posture of the user is the abnormal sitting posture may include determining whether the posture of the user corresponds to the sitting posture using the first frame angle value and the first rotation angle value, determining whether the first rotation angle value falls within a first angle range when determining that the posture of the user corresponds to the sitting posture, and determining that the sitting posture is the abnormal sitting posture when determining that the first rotation angle value is outside the first angle range.

The generating of the first torque may include generating the first torque in the first rotation direction through the driving module when determining that the first rotation angle value is outside the first angle range.

The providing of the external force may include providing the external force pushing the lower back area by rotating the lumbar support module in the first rotation direction based on the first torque.

In a state in which the first torque is generated, the method of operating the wearable apparatus may further include obtaining a second rotation angle value of the lumbar support module using an IMU of the wearable apparatus, determining whether the second rotation angle value corresponds to a first target angle value, and preventing the driving module from generating the first torque or generating a torque with an intensity less than the intensity of the generated first torque when determining that the second rotation angle value corresponds to the first target angle value.

The method may further include generating a second torque in a second rotation direction when rotation angle values of the lumbar support module, determined after the second rotation angle value, maintain a state corresponding to the first target angle value during a first time.

The first frame may include a first thigh frame corresponding to a first thigh of the user and a second thigh frame corresponding to a second thigh of the user.

The method may include calculating an average value of a first angle value, which is obtained by sensing the angle of the first thigh frame, and a second angle value, which is obtained by sensing the angle of the second thigh frame, determining a third angle value by subtracting the first rotation angle value from the calculated average value and determining whether the determined third angle value falls within a second angle range, and determining that the posture of the user corresponds to the sitting posture when the determined third angle value falls within the second angle range.

The method may further include determining whether the execution time of a first mode, in which the external force is provided to the user, elapses a second time and providing a notification to the user when determining that the execution time elapses the second time.

Each embodiment herein may be used in combination with any other embodiment(s) described herein.

The embodiments described herein may be implemented using a hardware component, a software component, and/or a combination thereof. A processing device may be implemented using one or more general-purpose or special-purpose computers, such as, for example, a processor, a controller and an arithmetic logic unit (ALU), a digital signal processor (DSP), a microcomputer, a field-programmable gate array (FPGA), a programmable logic unit (PLU), a microprocessor, or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is singular; however, one of ordinary skill in the art will appreciate that a processing device may include multiple processing elements and multiple types of processing elements. For example, the processing device may include a plurality of processors, or a single processor and a single controller. In addition, different processing configurations are possible, such as parallel processors.

The software may include a computer program, a piece of code, an instruction, or one or more combinations thereof, to independently or uniformly instruct or configure the processing device to operate as desired. Software and data may be stored in any type of machine, component, physical or virtual equipment, or computer storage medium or device capable of providing instructions or data to or being interpreted by the processing device. The software also may be distributed over network-coupled computer systems so that the software is stored and executed in a distributed fashion. The software and data may be stored by one or more non-transitory computer-readable recording mediums.

The methods according to the above-described embodiments may be recorded in non-transitory computer-readable media including program instructions to implement various operations of the above-described embodiments. The media may also include, alone or in combination with the program instructions, data files, data structures, and the like. The program instructions recorded on the media may be those specially designed and constructed for the purposes of embodiments, or they may be of the kind well-known and available to those having skill in the computer software arts. Examples of non-transitory computer-readable media include magnetic media such as hard disks, floppy disks, and magnetic tape; optical media such as CD-ROM discs and DVDs; magneto-optical media such as optical discs; and hardware devices that are specially configured to store and perform program instructions, such as read-only memory (ROM), random access memory (RAM), flash memory, and the like. Examples of program instructions include both machine code, such as produced by a compiler, and files containing higher-level code that may be executed by the computer using an interpreter.

The above-described hardware devices may be configured to act as one or more software modules in order to perform the operations of the above-described embodiments, or vice versa.

As described above, although the embodiments have been described with reference to the limited drawings, one of ordinary skill in the art may apply various technical modifications and variations based thereon. For example, suitable results may be achieved if the described techniques are performed in a different order, and/or if components in a described system, architecture, device, or circuit are combined in a different manner, or replaced or supplemented by other components or their equivalents.

While the disclosure has been illustrated and described with reference to various embodiments, it will be understood that the various embodiments are intended to be illustrative, not limiting. It will further be understood by those skilled in the art that various changes in form and detail may be made without departing from the true spirit and full scope of the disclosure, including the appended claims and their equivalents. It will also be understood that any of the embodiment(s) described herein may be used in conjunction with any other embodiment(s) described herein.

Therefore, other implementations, other embodiments, and equivalents of the claims are within the scope of the following claims.

## Claims

1. A wearable apparatus (500) comprising:
a driving module (540) comprising a motor and/or circuitry;
a first frame (530) configured for corresponding to a portion of a lower body of a user;
an angle sensor (520) configured to sense an angle of the first frame and obtain a first frame angle value;
a lumbar support module (550), comprising a lumbar support, connected to the driving module and configured to be positioned on and/or to support a lower back area of the user;
an inertial measurement unit (IMU) sensor (560); and
at least one processor (510) comprising processing circuitry,
wherein at least one processor is individually and/or collectively configured to obtain the first frame angle value via at least the angle sensor, obtain a first rotation angle value of the lumbar support module via at least the IMU sensor,
determine whether a sitting posture of the user is an abnormal sitting posture based on the first rotation angle value and the first frame angle value, and
control the driving module to cause the driving module to generate a first torque based on determining that the sitting posture is the abnormal sitting posture,
wherein the lumbar support module is configured to provide an external force
to the user via a rotational motion based on the generated first torque.

2. The wearable apparatus of claim 1, wherein at least one processor is individually and/or collectively configured to determine whether a posture of the user corresponds to the sitting posture using at least the first frame angle value and the first rotation angle value, determine whether the first rotation angle value falls within a first angle range when determining that the posture of the user corresponds to the sitting posture, and determine that the sitting posture is the abnormal sitting posture when determining that the first rotation angle value is outside the first angle range.

3. The wearable apparatus of claim 2, wherein
at least one processor is individually and/or collectively configured to control the driving module so that the driving module generates the first torque in a first rotation direction when it is determined that the first rotation angle value is outside the first angle range, and
the lumbar support module is configured to provide the external force pushing the lower back area by performing a rotational motion in the first rotation direction based on the first torque.

4. The wearable apparatus of claim 1, wherein at least one processor is individually and/or collectively configured to, in a state in which the first torque is generated, determine a second rotation angle value of the lumbar support module via at least the IMU sensor, determine whether the second rotation angle value corresponds to a first targe angle value, and perform, when it is determined that the second rotation angle value corresponds to the first target angle value, an operation of preventing the driving module from generating the first torque and/or an operation of controlling the driving module so that the driving module generates a torque with an intensity less than an intensity of the generated first torque.

5. The wearable apparatus of claim 4, wherein at least one processor is individually and/or collectively configured to control the driving module so that the driving module generates a second torque in a second rotation direction when rotation angle values of the lumbar support module, determined after the second rotation angle value, are maintained in a state corresponding to the first target angle value during a first time.

6. The wearable apparatus of claim 5, wherein at least one processor is individually and/or collectively configured to, in a state in which the second torque is generated, determine a third rotation angle value of the lumbar support module via at least the IMU sensor, determine whether the third rotation angle value corresponds to a second target angle value, and perform, when it is determined that the third rotation angle value corresponds to the second target angle value, an operation of preventing the driving module from generating the second torque and/or an operation of controlling the driving module so that the driving module generates a torque with an intensity less than an intensity of the second torque.

7. The wearable apparatus of claim 1, wherein
the first frame comprises a first thigh frame configured for corresponding to a first thigh of the user and a second thigh frame configured for corresponding to a second thigh of the user, and
at least one processor is individually and/or collectively configured to calculate an average value of a first angle value, which is obtained by sensing an angle of the first thigh frame, and a second angle value, which is obtained by sensing an angle of the second thigh frame, determine a third angle value at least by subtracting the first rotation angle value from the calculated average value, determine whether the determined third angle value falls within a second angle range, and determine that a posture of the user corresponds to the sitting posture when the determined third angle value falls within the second angle range.

8. The wearable apparatus of claim 1, further comprising:
a second frame (20) configured to connect the driving module to the lumbar support module and to be rotated by the generated first torque,
wherein the lumbar support module is configured to perform the rotational motion as the second frame is rotated by the generated first torque.

9. The wearable apparatus of claim 1, wherein at least one processor is individually and/or collectively configured to determine a magnitude value of the first torque based on at least one of the first rotation angle value and a magnitude value related to a torque magnitude received from an electronic device wirelessly connected to the wearable apparatus and control the driving module such that the first torque of the determined magnitude value is generated.

10. The wearable apparatus of claim 1, wherein at least one processor is individually and/or collectively configured to determine whether an execution time of a first mode, in which the external force is provided to the user, elapses a second time and, when it is determined that the execution time elapses the second time, control the wearable apparatus so that a notification is provided to the user.

11. The wearable apparatus of claim 10, wherein,
when it is determined that the execution time elapses the second time, at least one processor is individually and/or collectively configured to control the driving module so that the first frame connected to the driving module repeatedly performs a rotational motion in a first rotation direction and a rotational motion in a second rotation direction, and
the first frame is configured to repeatedly provide the portion of the lower body with an external force corresponding to the notification by repeatedly performing the rotational motion in the first rotation direction and the rotational motion in the second rotation direction.

12. The wearable apparatus of claim 1, wherein
at least one processor is individually and/or collectively configured to operate in a second mode in which the first torque and a second torque are alternately generated, and
the lumbar support module is configured to alternately perform, in the second mode, a rotational motion in a first rotation direction based on the generated first torque and a rotational motion in a second rotation direction based on the second torque.

13. The wearable apparatus of claim 12, wherein at least one processor is individually and/or collectively configured to determine a magnitude value of at least one of the first torque and the second torque based on the first rotation angle value, determine a time interval value for a duration time of at least one of the first torque and the second torque, and control the driving module so that the first torque and the second torque are alternately generated based on the determined magnitude value and the determined time interval value.

14. A method of operating a wearable apparatus (500), the method comprising:
obtaining a first frame angle value by sensing an angle of a first frame (530) corresponding to a portion of a lower body of a user;
obtaining a first rotation angle value of a lumbar support module (550) of the wearable apparatus, the lumbar support module comprising a lumbar support;
determining whether a sitting posture of the user is an abnormal sitting posture based on the first rotation angle value and the first frame angle value;
generating a first torque through a driving module (540) of the wearable apparatus when it is determined that the sitting posture is the abnormal sitting posture, wherein the driving module comprises a motor and/or circuitry; and providing an external force to the user by rotating the lumbar support module
connected to the driving module based on the generated first torque.

15. The method of claim 14, wherein the determining of whether the sitting posture of the user is the abnormal sitting posture comprises:
determining whether a posture of the user corresponds to the sitting posture using the first frame angle value and the first rotation angle value;
determining whether the first rotation angle value falls within a first angle range when it is determined that the posture of the user corresponds to the sitting posture; and
determining that the sitting posture is the abnormal sitting posture when it is determined that the first rotation angle value is outside the first angle range.
